# EUROPEAN PATENT APPLICATION

(11) **EP 4 512 411 A2**
(43) Date of publication of application: **26.02.2025**
(21) Application number: 23785029.2
(22) Date of filing: 06.04.2023
(51) Int. Cl.: A61K 38/17, A61K 47/64, A61K 47/65, A61K 47/55, A61P 35/00, C07K 14/435, A61K 47/68, C12Q 1/6886, G01N 33/574, G01N 33/68

(54) **ITGB2-MEDIATED DRUG DELIVERY SYSTEM**

(30) Priority: 07.04.2022 KR 20220043200; 07.04.2022 KR 20220043573
(71) Applicant: Twinpig Biolab, Inc., Seoul 02447 (KR)
(72) Inventor: BAE, Hyunsu, Seoul 05229 (KR); HAN, Ik-Hwan, Namyangju-si Gyeonggi-do 12160 (KR); KANG, Moonkyu, Namyangju-si Gyeonggi-do 12257 (KR); LEE, Heekyung, Yongin-si Gyeonggi-do 16927 (KR); CHOI, Jeongyoon, Seoul 01775 (KR); CHOI, Ilseob, Seoul 05112 (KR); YANG, Juwon, Seoul 02451 (KR); CHOI, Hongseo, Seoul 04999 (KR); SHIN, Jin Sun, Seoul 01854 (KR)
(74) Representative: Isarpatent
(86) International application number: PCT/KR2023/004678
(87) International publication number: WO 2023/195805

(57) **Abstract**

The present invention relates to a drug delivery conjugate that specifically binds to integrin beta 2 (ITGB2) on M2 tumor-associated macrophages. The drug delivery conjugate comprising an ITGB2-binding moiety allowing specific binding to ITGB2, of the present invention, selectively (or specifically) acts on the M2 tumor-associated macrophages, thereby reducing the side effects of a drug and increasing the efficacy thereof, and thus can be effectively used as a drug delivery conjugate for apoptosis-inducing agents, anticancer agents, contrast agents, and the like.

## Description

### [Technical Field]

The present disclosure relates to a drug delivery conjugate that specifically binds to ITGB2.

### [Background Art]

Conventional anti-cancer therapies have been studied to directly attack cancer cells or enhance the activity of immune cells in the body that directly attack cancer cells. However, these anti-cancer drugs also attack other normal cells other than cancer cells, resulting in many side-effects such as hair loss, nausea, and vomiting, and cause additional reactions due to an excessive increase of immune cells. Compared to existing chemotherapy or radiation therapy, anticancer immunotherapy is a method of treating cancer by using an in vivo immune system capable of minimizing side effects. Among these anticancer immunotherapy techniques, there have been actively progressed cell therapy methods that activate therapeutic immune cells such as T cells (including CAR-T), dendritic cells, and natural killer cells outside the body and then directly inject the activated cells into the body, anticancer vaccine methods that directly activate immune cells existing in the body by injecting cancer antigens and immune-activating substances into the body, thereby increasing anticancer efficacy, etc. However, these cell therapy agents and cancer vaccines are mainly used for blood cancer-related diseases, and have a disadvantage of having very low therapeutic efficacy in most solid cancers. One of these reasons is due to microenvironmental factors that suppress the immune function around solid cancers. In fact, cells that lower the function of immune cells in the tumor microenvironment (MDSC: myeoloid-derived stromal cells, Treg: regulatory T cells, TAM: tumor-associated macrophages), immunosuppressive cytokines, and metabolites are actively working, which rapidly reduces the activity of immune-activating substances and therapeutic immune cells. Therefore, it has been important to develop a therapeutic agent having an anticancer effect by controlling only the surrounding microenvironment of the tumor cells without directly affecting tumor cells and immune cells to block nutrient supply to tumor cells and angiogenesis around the tumor cells. The tumor microenvironment is greatly considered as a therapeutic target by contributing to the proliferation and survival of malignant cells, angiogenesis, metastasis, abnormally adaptive immunity, and reduced responses to hormones and chemotherapeutic agents.

The microenvironment around the tumor consists of endothelial cells, inflammatory cells, and fibroblasts, and in the 1970s, it was found that tumor-associated macrophages (TAMs) play an important role in tumor growth. Tumor-associated macrophages play an important role in the overall tumor microenvironment, including cancer growth and metastasis, and are classified into two phenotypes of tumor-suppressive M1-type macrophages and tumor-supportive M2-type macrophages. The M1-type macrophages have a strong antigen-presenting ability, are generally activated by interferon-γ, lipopolysaccharide (LPS), and tumor necrosis factor (TNF)-α, and have proinflammatory and bactericidal effects. The M2-type macrophages are known to promote immunosuppression, tumorigenesis, and angiogenesis by releasing various extracellular matrix components, angiogenesis and chemotaxis factors. Generally, the M2-type macrophages are induced by IL-4 and IL-13, and are distinguished from M1-type tumor-associated macrophages by expressing markers such as arginase-1, mannose (MMR, CD206), scavenger receptors (SR-A, CD204), CD163, and IL-10. Tumor-associated macrophages present around tumors are closely related to the growth and metastasis of tumor cells, and it has been reported that when a large number of M2-type tumor-associated macrophages exist around tumors in cancer patients, the patient's prognosis and survival rate are poor. It has been reported that the M2-type macrophages produce cytokines such as IL-10, TGFβ, and CCL18 that promote cancer growth, and receptors such as PDL1 and B7-1/2 present on the surface of the M2-type tumor-associated macrophages suppress the antitumor activity of T cells and NK cells. Since tumor growth, differentiation, and metastasis occur actively in a microenvironment where the M2-type tumor-associated macrophages exist in large numbers, it is necessary to develop targeted therapeutic agents targeting M2-type tumor-associated macrophages.

### [Disclosure]

### [Technical Problem]

An object of the present disclosure is to provide a drug delivery conjugate targeting M2 macrophages.

In addition, another object of the present disclosure is to provide a pharmaceutical composition for preventing or treating cancer.

In addition, yet another object of the present disclosure is to provide a method for screening a substance that specifically delivers a drug to M2 tumor-associated macrophages.

In addition, yet another object of the present disclosure is to provide a method for selecting a patient having responsiveness to an anticancer agent targeting M2 macrophages.

In addition, yet another object of the present disclosure is to provide a method for treating cancer.

In addition, yet another object of the present disclosure is to provide a use of a drug delivery conjugate used for the preparation of a pharmaceutical composition for preventing or treating cancer.

### [Technical Solution]

In order to achieve the above aspects, the present disclosure provides a drug delivery conjugate including an ITGB2-binding moiety; and a drug binding to the ITGB2-binding moiety.

In addition, the present disclosure provides a pharmaceutical composition for preventing or treating cancer.

In addition, the present disclosure provides a method for screening a substance that specifically delivers a drug to M2 tumor-associated macrophages.

In addition, the present disclosure provides a method for selecting a patient having responsiveness to an anticancer agent targeting M2 macrophages.

In addition, the present disclosure provides a method for treating cancer including administering a drug delivery conjugate of the present disclosure in a pharmaceutically effective amount to a subject suffering from cancer.

In addition, the present disclosure provides a use of a drug delivery conjugate of the present disclosure used for the preparation of a pharmaceutical composition for preventing or treating cancer.

### [Advantageous Effects]

According to the present disclosure, it was confirmed that Integrin beta 2 (ITGB2) is a specific marker for M2 macrophage-mediated cancer, and the drug delivery conjugate including an ITGB2-binding moiety that specifically binds to ITGB2, of the present disclosure, selectively (or specifically) acts on only the M2 tumor-associated macrophages, thereby reducing the side effects of a drug and increasing the efficacy thereof, and thus may be effectively used as a drug delivery conjugate for apoptosis-inducing agents, anticancer agents, contrast agents, and the like.

### [Description of Drawings]

FIG. 1A is a diagram showing the affinity of TAMpep for M2 macrophages confirmed by flow cytometry.
FIG. 1B is a graph quantifying the affinity of TAMpep for M2 macrophages (**P* < 0.05 versus the M0.).
FIG. 2A is an analytical Venn diagram of M2/M0 and M1/M0 ratios, which is a diagram identifying proteins binding to TAMpep in M2 macrophages.
FIG. 2B is a diagram identifying proteins binding to TAMpep in M2 macrophages by a scatter plot analysis of a M2/M0 ratio.
FIG. 2C is a diagram identifying proteins binding to TAMpep in M2 macrophages by a scatter plot analysis of a M1/M0 ratio.
FIG. 2D is a diagram identifying proteins binding to TAMpep in M2 macrophages by a scatter plot analysis of a M2/M1 ratio.

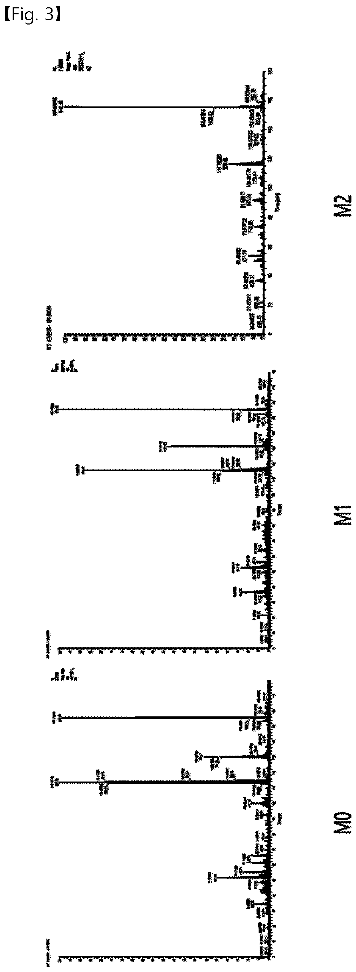

FIG. 3 is a diagram illustrating LC-MS chromatogram data obtained by reacting TAMpep826-biotin with cell membrane proteins extracted from M0, M1, and M2 macrophages, respectively.
FIG. 4 is a diagram showing identification of target proteins of TAMpep826 using LC-MS/MS proteomics.
FIG. 5 is a diagram showing quantitative RT-PCR conditions.
FIG. 6 is a diagram confirming ITGB2 mRNA expression in M2 macrophages.
FIG. 7 is a diagram confirming ITGB2 protein expression in M2 macrophages.
FIG. 8 is a diagram confirming ITGB2 protein expression in M2 macrophages by immunofluorescence analysis.
FIG. 9 is a diagram confirming ITGB2 expression on the cell membrane of M2 macrophages.
FIG. 10 is a diagram confirming interaction between TAMpep826 and ITGB2 in M2 macrophages by pull-down assay.
FIG. 11 is a diagram confirming interaction between TAMpep826 and ITGB2 in M2 macrophages by immunofluorescence analysis.
FIGS. 12 and 13 are diagrams confirming binding sites of TB511 and ITGB2 through docking simulation.
FIG. 14 is a schematic diagram showing that an anti-CD18 antibody used in the fabrication of an ITGB2 binding ADC of the present disclosure specifically binds to an active conformation (extended-open form) of CD18 in M2 TAM.
FIG. 15 is a diagram showing ITGB2 immobilized on the surface of a sensor chip.
FIG. 16 is a diagram confirming binding affinity between anti-ITGB2 antibody and ITGB2 protein by SPR.
FIG. 17 is a diagram confirming binding affinity between Melittin-dKLA and ITGB2 protein by SPR.
FIG. 18 is a diagram confirming the mRNA expression of ITGB2 in an ITGB2 expression suppression cell model established through Crispr/cas9 in M2 macrophages.
FIG. 19 is a diagram confirming an apoptosis reduction effect of Melittin-dKLA by suppressing ITGB2 expression in M2 macrophages.
FIG. 20 is a diagram confirming an apoptosis-induction reduction effect of TB511 (TAMpep826-dKLA) by suppressing ITGB2 expression in M2 macrophages.
FIG. 21 is a diagram confirming an apoptosis-induction reduction effect of TB511 by an ITGB2 antibody in M2 macrophages.
FIGS. 22 and 23 are diagrams confirming effects of TB511 in breast cancer 3D organoids.
FIGS. 24 and 25 are diagrams confirming effects of TB511 in lung cancer 3D organoids.
FIG. 26 is a diagram confirming an effect of M-DM1 in lung cancer 3D organoids.
FIGS. 27 and 28 are diagrams confirming effects of TB511 in anticancer drug-resistant lung cancer 3D organoids.
FIGS. 29 and 30 are diagrams confirming effects of TB511 in anticancer drug-resistant prostate cancer 3D organoids.
FIG. 31 is a diagram confirming an effect of CD18-DM1 in breast cancer 3D organoids.
FIG. 32 is a diagram confirming an effect of CD18-MMAE in breast cancer 3D organoids.
FIGS. 33 and 34 are diagrams confirming effects of ADCs (CD18-DM1 and CD18-MMAE) in lung cancer 3D organoids.
FIGS. 35 and 36 are diagrams confirming effects of ADCs (CD18-DM1 and CD18-MMAE) in anticancer drug-resistant lung cancer 3D organoids.
FIGS. 37 and 38 are diagrams confirming effects of ADCs (CD18-DM1 and CD18-MMAE) in anticancer drug-resistant prostate cancer 3D organoids.

### [Best Mode of the Invention]

Hereinafter, embodiments of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used in the present disclosure are terminologies used to properly express preferred embodiments of the present disclosure, which may vary according to a user, an operator's intention, or customs in the art to which the present disclosure pertains. Therefore, these terminologies used herein will be defined based on the contents throughout the specification. Throughout the specification, unless explicitly described to the contrary, when a certain part "comprises" a certain component, it will be understood to further include another component but not the exclusion of other components.

All technical terms used in the present disclosure, unless otherwise defined, are used as the meaning as commonly understood by those skilled in the related art of the present disclosure. In addition, although preferred methods and samples are described in the present disclosure, similar or equivalent methods and samples thereto are also included in the scope of the present disclosure. The contents of all publications disclosed as references in this specification are incorporated in the present disclosure.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned in the present disclosure as abbreviations are also described as follows according to the IUPAC-IUB nomenclature.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In one aspect, the present disclosure relates to a drug delivery conjugate including an Integrin beta 2 (ITGB2)-binding moiety; and a drug binding to the ITGB2-binding moiety.

In one embodiment, the ITGB2 may include an amino acid sequence represented by SEQ ID NO: 7 or 8.

In one embodiment, the ITGB2-binding moiety may specifically bind to an ITGB2 protein present on the cell membrane of M2 tumor-associated macrophages and may include a small molecule, a virus, an antibody, an antibody fragment, an aptamer, a hormone, a cytokine, a chemokine, a ligand, a peptide that is a portion of a cytokine, or a peptide that is a portion of a ligand, that specifically binds to the ITGB2.

In one embodiment, the ITGB2-binding moiety may further include a targeting sequence, a tag, a labeled residue or an amino acid sequence designed for a specific purpose to increase the half-life or stability.

In one embodiment, the ITGB2-binding moiety may specifically bind to an active conformation in which CD18 in an extended.

In one embodiment, the ITGB2-binding moiety may bind to amino acids at positions 449 to 617 of ITGB2.

In one embodiment, the ITGB2-binding moiety may bind to amino acids at positions 466 to 565 of ITGB2.

In one embodiment, the ITGB2-binding moiety may include melittin, a variant thereof or analogues thereof and may include an amino acid sequence represented by SEQ ID NO: 1 or 2.

In one embodiment, the drug delivery conjugate may include at least one selected from the group consisting of amino acid sequences represented by SEQ ID NOs: 3 to 6.

In one embodiment, the drug delivery conjugate may be a peptide-drug conjugate (PDC), Melittin-dKLA including an amino acid sequence represented by SEQ ID NO: 5, TB511 including an amino acid sequence represented by SEQ ID NO: 6, and M-DM1 in which a drug DM1 is conjugated to maleimide-modified melittin.

In one embodiment, the TB511 may bind to amino acids at positions 466 to 565 of ITGB2, LEU(6) of TB511 may bind to LEU(528), THR(538), LEU(556), CYS(557) and PHE(558) sites of ITGB2, and TRP(12) of TB511 may bind to GLU(466), ILE(469), CYS(470) and ARG(471) of ITGB2.

In one embodiment, the ITGB2-binding moiety may bind to an epitope including amino acids at positions 732 to 748 of ITGB2 (CD18) (SEQ ID NO: 9), an epitope including amino acids at positions 504 to 508, an epitope including amino acids at positions 534 to 546, an epitope including amino acids at positions 449 to 617, or an epitope including amino acids at positions 23 to 700 (Gln23-Asn700).

In one embodiment, the ITGB2-binding moiety may be an antibody or immunologically active fragment thereof that binds to the epitope.

In one embodiment, the immunologically active fragment may be any one selected from the group consisting of Fab, Fd, Fab', dAb, F(ab'), F(ab')₂, a single chain fragment variable (scFv), Fv, a single chain antibody, a Fv dimmer, a complementarity determining region fragment, a humanized antibody, a chimeric antibody, and a diabody.

In one embodiment, the drug delivery conjugate may be an antibody-drug conjugate (ADC).

In one embodiment, the ITGB2-binding moiety of the drug delivery conjugate and the drug may be covalently linked via a linker or non-covalently linked without the linker.

In one embodiment, the ITGB2-binding moiety of the drug delivery conjugate and the drug may be linked/conjugated via a linker, and the linker may be any linker having a functional group that may bind via an amine group, carboxyl group, or sulfhydryl group of a protein such as a peptide, a ligand, an antibody, or an antibody fragment, or a phosphate group or hydroxyl group of a nucleic acid such as an aptamer.

In one embodiment, the linker may be selected and used appropriately depending on the drug. For example, a linker having an aldehyde reactive group may be linked to a drug and bound to an amino group at the N-terminus of an antibody (cell targeting region), which is the ITGB2-binding moiety of the drug delivery conjugate.

The functional group of the linker may be isothiocyanate, isocyanates, acyl azide, NHS ester, sulfonyl chloride, aldehyde, glyoxal, epoxide, oxirane, carbonate, arylhalide, imidoester, carbodiimide, anhydride, fluorophenyl ester, hydroxymethyl phosphine, maleimide, haloacetyl, pyridyldisulfide, thiosulfonate, vinylsulfone, or the like. The linker may be a linker that is cleavable by a protease, cleavable under acid or basic conditions, cleavable by high temperature or light irradiation, or cleavable under reducing or oxidizing conditions, or a linker that is not cleavable under these conditions. Examples of the cleavable linker may include hydrazone linkers that are cleaved under acidic conditions, peptide linkers that are cleaved by proteases, linkers having a disulfide functional group that are cleaved under reducing conditions, and the like. Non-cleavable linkers may include Maleimidomethyl cyclohexane-1-carboxylate (MCC) linkers, maleimidocaproyl (MC) linkers, or derivatives thereof, such as succinimidyl-4-(N-maleimidomethyl)cyclohexane-1-carboxylate (sMCC) linkers or sulfosuccinimidyl-4-(N-maleimidomethyl)cyclohexane-1-carboxylate (sulfo-sMCC).

In addition, the linker may be a self-immolative linker or a traceless linker. The self-immolative linker may include, for example, linkers disclosed in U.S. Pat. No. 9,089,614 entitled "Hydrophilic self-immolative linkers and conjugates thereof", and linkers disclosed in International Publication No. WO2015038426 entitled "SELF-IMMOLATIVE LINKERS CONTAINING MANDELIC ACID DERIVATIVES, DRUG-LIGAND CONJUGATES FOR TARGETED THERAPIES AND USES THEREOF", and the traceless linker may include a phenylhydrazide linker, an aryl-triazene linker, linkers disclosed in the literature [Blaney, et al., "Traceless solid-phase organic synthesis", Chem Rev. 102: 2607-2024 (2002)], and the like.

In addition to the linkers exemplified above, numerous linkers applicable to the present disclosure are known in the art through a considerable number of documents. Such a literature may specifically refer to the literatures [Castaneda, et al, "Acidcleavable thiomaleamic acid linker for homogeneous antibodydrug conjugation", Chem Commun. 49: 8187-8189 (2013)], [Lyon, et al, "Self-hydrolyzing maleimides improve the stability and pharmacological properties of antibody-drug conjugates", Nat Biotechnol. 32(10):1059-1062 (2014)], [Dawson, et al "Synthesis of proteins by native chemical ligation", Science 1994, 266, 776-779], [Dawson, et al "Modulation of Reactivity in Native Chemical Ligation through the Use of Thiol Additives", J Am Chem Soc. 1997, 119, 4325-4329], [Hackeng, et al "Protein synthesis by native chemical ligation: Expanded scope by using straightforward methodology", Proc Natl Acad Sci USA 1999, 96, 10068-10073], [Wu, et al "Building complex glycopeptides: Development of a cysteine-free native chemical ligation protocol", Angew Chem Int Ed 2006, 45, 4116-4125], [Geiser et al "Automation of solid-phase peptide synthesis" in Macromolecular Sequencing and Synthesis, Alan R Liss, Inc, 1988, pp 199-218], [Fields, G and Noble, R (1990) "Solid phase peptide synthesis utilizing 9-fluoroenylmethoxycarbonyl amino acids", Int J Peptide Protein Res 35:161-214], etc., and US Patent No. 6,884,869, US Patent No. 7,498,298, US Patent No. 8,288,352, US Patent No. 8,609,105, US Patent No. 8,697,688, US Patent Publication No. 2014/0127239, US Patent Publication No. 2013/028919, US Patent Publication No. 2014/286970, US Patent Publication No. 2013/0309256, US Patent Publication No. 2015/037360, US Patent Publication No. 2014/0294851, International Patent Publication No. WO2015057699, International Patent Publication No. WO2014080251, International Patent Publication No. WO2014197854, International Patent Publication WO2014145090, International patent publication WO2014177042, etc.

The ITGB2-binding moiety of the drug delivery conjugate of the present disclosure and the drug may also be bound via a biocompatible polymer or using the biocompatible polymer as a carrier. The biocompatible polymer means a polymer that has tissue compatibility and blood compatibility, which does not cause tissue necrosis or blood coagulation when in contact with living tissue or blood.

A synthetic polymer as the biocompatible polymer includes polyester, polyhydroxyalkanoate (PHA), poly(α-hydroxyacid), poly(β-hydroxyacid), poly(3-hydroxybutyrate-co-valerate (PHBV), poly(3-hydroxyproprionate (PHP), poly(3-hydroxyhexanoate (PHH), poly(4-hydroxyacid), poly(4-hydroxybutyrate), poly(4-hydroxyvalerate), poly(4-hydroxyhexanoate), poly(esteramide), polycaprolactone, polylactide, polyglycolide, poly(lactide-co-glycolide) (PLGA), polydioxanone, polyorthoester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, ethylene vinyl alcohol copolymer (EVOH), polyurethane, silicone, polyester, polyolefin, polyisobutylene and ethylene-alphaolefin copolymer, styrene-isobutylene-styrene triblock copolymer, acrylic polymer and copolymer, vinyl halide polymer and copolymer, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatics, polystyrene, polyvinyl ester, polyvinyl acetate, ethylene-methyl methacrylate copolymer, acrylonitrile-styrene copolymer, ABS resin and ethylenevinyl acetate copolymer, polyamide, alkyd resin, polyoxymethylene, polyimide, polyether, polyacrylate, polymethacrylate, polyacrylic acid-co-maleic acid or polyaminoamine, and the natural polymer is chitosan, dextran, cellulose, heparin, hyaluronic acid, alginate, inulin, starch or glycogen.

The linker used to bind the ITGB2-binding moiety and the drug is preferably a linker that is stable outside a target cell and is not cleaved, and is not cleaved even in acidic conditions inside the target cell, such as endosome or lysosome. The linker is stable outside the target cell and is not cleaved, allowing the drug to be transported into the cell, and is not cleaved in the acidic conditions, such as the endosome or lysosome, allowing the drug to be transported from the endosome or lysosome to the cytoplasm.

In the drug delivery conjugate of the present disclosure, the drug may also be noncovalently bound to the ITGB2-binding moiety. For example, intercalator agents, such as doxorubicin, which is an anticancer agent that is intercalated into nucleic acids to exhibit an effect, may be noncovalently bound to aptamers through intercalation when the aptamers are used as a cell targeting region of the drug delivery conjugate. Since the aptamer is an oligonucleotide molecule, there is base stacking of nucleotide bases, and the drug may bind between these base stacks through intercalation.

In one embodiment, the ITGB2-binding moiety may be further conjugated to an RNA, a DNA, an antibody, an effector, a drug, a prodrug, a toxin, a peptide or a delivery molecule (see Shoari et al., Pharmaceutics 13:1391, pp. 1-32 (2021)).

In the drug delivery conjugate of the present disclosure, the drug is not particularly limited as long as it is a drug that is transported into the cell to exhibit an effect. Such a drug may be a drug formed of any small molecule compound, such as a cytotoxic anticancer agent and the like, and any biopharmaceutical such as a recombinant protein, siRNA, and the like. In addition, in terms of the efficacy, the drug may be an anti-inflammatory agent, an analgesic, an anti-arthritic, an antispasmodic, an antidepressant, an antipsychotic, a tranquilizer, an anxiolytic, a narcotic antagonist, an anti-Parkinson's disease agent, a cholinergic agonist, an anticancer agent, an angiogenesis inhibitor, an immunosuppressant, an immunostimulant, an antiviral drug, an antibiotic, appetite suppressant, an analgesic, an anticholinergic, an antihistamine, an antimigraine, a hormonal agent, a coronary vasodilator, a vasodilator, a contraceptive, an antithrombotic, a diuretic, an antihypertensive agent, a cardiovascular disease therapeutic agent, diagnostic agents such as contrast agents, and the like.

In one embodiment, the drug may be a compound, RNA, DNA, an antibody, an effector, a prodrug, a toxin, a peptide, or a radionuclide.

In one embodiment, the drug may be immunogenic apoptosis-inducing agents, pro-apoptotic peptides, microtubulin structure formation inhibitors, meiosis inhibitors, topoisomerase inhibitors, DNA intercalators, toxins or anticancer agents.

In one embodiment, the pro-apoptotic peptide may be selected from the group consisting of KLA, alpha-defensin-1, BMAP-28, Brevenin-2R, Buforin IIb, cecropin A-Magainin 2 (CA-MA-2), Cecropin A, Cecropin B, chrysophsin-1, D-K6L9, Gomesin, Lactoferricin B, LLL27, LTX-315, Magainin 2, Magainin II-bombesin conjugate (MG2B), Pardaxin, and combinations thereof.

In one embodiment, the immunogenic apoptosis-inducing agent may be selected from the group consisting of an anthracycline-based anticancer agent, a taxane-based anticancer agent, an anti-EGFR antibody, a BK channel agonist, bortezomib, cardiac glycoside, cyclophosmide-based anticancer agents, a GADD34/PP1 inhibitor, LV-tSMAC, Measles virus, bleomycin, mitoxantrone, oxaliplatin, and combinations thereof.

In one embodiment, the anticancer agent may be selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), Streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof, and may be DM1 or MMAE.

In the present disclosure, the drug is preferably a cytotoxic anticancer agent. The cytotoxic anticancer agent may be broadly classified into antimetabolites, microtubulin targeting agents (tubulin polymerase inhibitor and tubulin depolymerisation), alkylating agents, antimitotic agents, DNA cleavage agents, DNA cross-linker agents, DNA intercalator agents, DNA topoisomerase inhibitors, and the like. As the antimetabolites, folic acid derivatives such as methotrexate, purine derivatives such as cladribine, pyrimidine derivatives such as azacitidine, doxifluridine, fluorouracil, etc. are known in the art. As the microtubule targeting agents, auristatin-based drugs such as monomethylauristatin E (MMAE), monomethylauristatin F (MMAF), dolastatin, maytansines, and the like are known in the art. As the alkylating agents, alkyl sulfonate agents such as busulfan and treosulfan, nitrogen mustard derivatives such as bendamustine, platinum agents such as cisplatin and heptaplatin, and the like are known in the art. In addition, as the antimitotic agents, taxane agents such as docetaxel and paclitaxel, vinca alkalids such as vinflunine, podophyllotoxin derivatives such as etoposide, and the like are known in the art. As the DNA cleavage agents, calicheamicins and the like are known in the art. As the DNA cross-linker agents, PBD dimer, and the like are known in the art. In addition, as the DNA intercalators, doxorubicin and the like are known in the art, and as the DNA topoisomerase inhibitors, SN-28 and the like are known in the art.

In addition, the drug may also be genes, plasmid DNA, antisense oligonucleotide, siRNA, peptides, ribozyme, viral particles, immunomodulators, proteins, contrast agents, etc. More specifically, the drug may also be a gene encoding Rb94 which is a variant of a retinoblastoma tumor suppressor gene, a gene encoding apoptin, which induces apoptosis only in tumor cells, may be an antisense oligonucleotide (sequence: 5'-TCC ATG GTG CTC ACT-3') for therapeutic targets such as HER-2, and may also be a diagnostic contrast agent such as a Gd-DTPA substance and the like, which are MRI contrast agents.

The complex of the drug delivery conjugate of the present disclosure and the drug may be prepared as a pharmaceutical composition in the form of an oral formulation or a parenteral formulation depending on a route of administration by a conventional method known in the art, including a pharmaceutically acceptable carrier. Herein, the term "pharmaceutically acceptable carrier" may refer to a carrier or a diluent which does not inhibit biological activity and properties of a compound to be administered without stimulating organisms. In the composition formulated with a liquid solution, the pharmaceutically acceptable carrier is suitable for sterilization and living bodies and may use saline, sterilized water, ringer's solution, buffered saline, albumin injection solution, dextrose solution, maltodextrin solution, glycerol, ethanol, and a mixture of at least one of these ingredients. If necessary, other general additives, such as antioxidants, buffers, bacteriostatic agents, and the like, may be added.

In one embodiment, the drug delivery conjugate of the present disclosure may specifically deliver the drug to M2 tumor-associated macrophages.

In the present disclosure, the ITGB2-binding moiety provides a targeting function by enabling selective binding through binding to ITGB2 of target cells, M2 tumor-associated macrophages. This ITGB2-binding moiety specifically binds to ITGB2 present on the surface of the target cell to induce endocytosis, thereby enabling the drug binding to the ITGB2-binding moiety to move into the cell.

In the present disclosure, antibodies, aptamers, hormones (e.g., erythropoietin hormone) secreted from specific cells and acting on surface receptors of other cells, which are target cells, thereby playing a role in signal transmission between cells, cytokines or chemokines (e.g., IL13), ligands that are biomolecules such as vascular endothelial growth factor (VEGF) and brain-derived neurotrophic factor (BDNF) that bind to the surface receptor of the target cell, and peptides that are partial regions of these factors having specific binding ability to receptors may be used as the ITGB2-binding moiety. Representatively, the antibody or the aptamer may be used, and the antibody as the ITGB2-binding moiety may be a monoclonal antibody, a polyclonal antibody, and a multispecific antibody (i.e., an antibody that recognizes two or more antigens or two or more epitopes, such as a bispecific antibody and the like), or any antibody that has the ability to specifically bind to ITGB2, such as an antibody fragment, a chemically modified antibody, a chimeric antibody (such as a humanmouse chimeric antibody, a human-monkey chimeric antibody), or a humanized antibody or human antibody with reduced immunogenicity. In addition, various forms of the antibody fragment and the chemically modified antibody are known in the art, and for example, may include Fab, Fd, Fab', dAb, F(ab'), F(ab')₂, single chain fragment variable (scFv), Fv, single-chain antibodies, Fv dimers, complementarity determining region fragments, humanized antibodies, chimeric antibodies, and diabodies, and antibody fragments obtained by treating antibodies with protein cleaving enzymes such as papain and pepsin.

With regard to methods for preparing antibodies, antibodies that have improved specificity or immunogenicity for target antigens by applying artificial modification to natural antibodies, etc., various documents known in the art, such as U.S. Pat. No. 4,444,887, U.S. Pat. No. 4,716,111, U.S. Pat. No. 5,545,806, U.S. Pat. No. 5,814,318, International Publication No. WO 98/46645, International Publication No. WO 98/50433, International Publication No. WO 98/24893, International Publication No. WO 98/16654, International Publication No. WO 96/34096, International Publication No. WO 96/33735, the literature [Protein Eng 1994, 7(6):805-814], the literature [Proc Natl Acad Sci U S A 1994, 91:969-973], etc. may be referred.

The aptamer as a cell targeting domain may be a single-stranded DNA aptamer or a single-stranded RNA aptamer. Like the antibody, the aptamer refers to a nucleic acid ligand that may specifically bind to a target molecule such as a target antigen, and if the aptamer may specifically bind to a target molecule, the aptamer may be a doublestranded DNA or RNA aptamer. Methods for preparing and selecting aptamers capable of specifically binding to such target molecules are all known in the art. Specific methods for screening aptamers or the use of appropriate reagents, materials, etc. refer to the literatures [Methods Enzymol 267:275-301, 1996], [Methods Enzymol 318:193-214, 2000], etc. The aptamer may be modified in sugars, phosphates and/or bases to enhance the in vivo half-life. Nucleotides modified in these sugars, phosphates and/or bases are specifically known in the art, including preparation methods thereof. For example, modified nucleotides in sugars may include nucleotides in which a hydroxyl group (OH group) of the sugar is modified with a halogen group, an aliphatic group, an ether group, an amine group, etc., nucleotides in which ribose or deoxyribose itself as the sugar may be replaced with sugar analogues such as α-anomeric sugars, epimeric sugars such as arabinose, xyloses, or lyxoses, pyranose sugars, and furanose sugars, and the like. In addition, for example, modifications in phosphate may include modification of phosphate into P(O)S(thioate), P(S)S(dithioate), P(O)NR2(amidate), P(O)R, P(O)OR', CO or CH2(formacetal). Here, the R or R' is H or substituted or unsubstituted alkyl, etc., and when modified in phosphate, the linking group becomes -O-, -N-, -S- or -C-, and adjacent nucleotides are bonded to each other through this linking group.

The drug delivery conjugate of the present disclosure specifically and directly binds to ITGB2 expressed on the cell membrane of M2 tumor-associated macrophages, particularly, to ITGB2 (CD18) in an active conformation in M2 TAM, thereby enabling the drug effect to be selectively exhibited only on M2 tumor-associated macrophages in which the target molecule ITGB2 is expressed and activated, without side effects in which the drug effect is non-selectively exhibited.

In one aspect, the present disclosure relates to a peptide-drug conjugate (PDC) including an ITGB2-binding moiety including melittin, a variant thereof or analogues thereof; and a drug binding to the ITGB2-binding moiety.

In one embodiment, the PDC may bind to amino acids at positions 466 to 565 of ITGB2, and may bind to LEU(528), THR(538), LEU(556), CYS(557), PHE(558), GLU(466), ILE(469), CYS(470) or ARG(471) of ITGB2.

In one embodiment, the PDC may be Melittin-dKLA including an amino acid sequence represented by SEQ ID NO: 5, TB511 including an amino acid sequence represented by SEQ ID NO: 6, and M-DM1 in which a drug DM1 is conjugated to maleimide-modified melittin.

In one embodiment, TB511 may bind to amino acids at positions 466 to 565 of ITGB2, LEU(6) of TB511 may bind to LEU(528), THR(538), LEU(556), CYS(557) and PHE(558) sites of ITGB2, and TRP(12) of TB511 may bind to GLU(466), ILE(469), CYS(470) and ARG(471) of ITGB2.

In one aspect, the present disclosure relates to an antibody-drug conjugate (ADC) including an antibody or immunologically active fragment thereof that binds to ITGB2; and a drug binding to the antibody or immunologically active fragment thereof.

In one embodiment, the ADC may bind to an epitope including amino acids at positions 732 to 748 of ITGB2, an epitope including amino acids at positions 504 to 508, an epitope including amino acids at positions 534 to 546, an epitope including amino acids at positions 449 to 617, or an epitope including amino acids at positions 23 to 700.

In one embodiment, the ADC may further include an antibody-drug conjugate (ADC) linker, and the ADC linker may be 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl) cyclohexane-1 carboxylate (SMCC), valine-citrulline-p-aminobenzyloxycarbonyl (val-cit-PAB), or N-succinimidyl (4-iodo-acetyl) aminobenzoate (SIAB).

In one embodiment, the antibody-drug conjugate may form a complex with an anti-CD18 antibody or immunologically active fragment thereof and a drug via an ADC linker.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment fabricated by gene fusion (WO94/13804).

The antibody or immunologically active fragment thereof of the present disclosure may be selected from the group consisting of animal-derived antibodies, chimeric antibodies, humanized antibodies, human antibodies, and immunologically active fragments thereof. The antibody may be fabricated recombinantly or synthetically.

Animal-derived antibodies fabricated by immunizing an animal to be immunized with a desired antigen may generally cause immune rejection when administered to humans for treatment, and chimeric antibodies have been developed to suppress such immune rejection. The chimeric antibody is obtained by substituting a constant region of an animal-derived antibody, which causes an anti-isotype response, with a constant region of a human antibody using a genetic engineering method. Compared to animal-derived antibodies, the chimeric antibody is improved significantly in the anti-isotype response, but includes side effects on potential anti-idiotypic responses because animal-derived amino acids are still present in a variable region. The humanized antibody is developed to improve these side effects. The humanized antibody is fabricated by grafting complementarity determining regions (CDRs), which play an important role in antigen binding in the variable regions of the chimeric antibody, to a human antibody framework.

In the CDR grafting technology for fabricating the humanized antibody, it is most important to select an optimized human antibody that may best accept the CDR region of the animal-derived antibody, and to this end, applications of an antibody database, analysis of a crystal structure, molecular modeling technology, and the like are used. However, even if the CDR region of the animal-derived antibody is grafted into an optimized human antibody framework, in some cases, there are amino acids that affect antigen binding while being located in the framework of the animal-derived antibody. As a result, since there are many cases in which the antigen-binding ability is not conserved, it may be required to apply additional antibody engineering techniques to restore the antigen-binding ability.

The antibody or the immunologically active fragment thereof may be isolated from a living body (not present in a living body) or may non-naturally occur, and for example, may be synthetically or recombinantly fabricated.

As used in the present disclosure, the "antibody" refers to a substance fabricated by stimulation of an antigen in the immune system, and the type thereof is not particularly limited, and may be obtained naturally or non-naturally (e.g., synthetically or recombinantly). The antibody is advantageous for mass expression and fabrication because of being very stable in vivo as well as ex vivo and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

As used in the present disclosure, the term "heavy chain" is interpreted as meaning including all of a full-length heavy chain including a variable region domain V_{H} including an amino acid sequence having a sufficient variable region sequence to impart specificity to an antigen, three constant region domains C_{H1}, C_{H2} and C_{H3} and hinges, and fragments thereof. In addition, the term "light chain" is interpreted as meaning including all of a full-length light chain including a variable region domain V_{L} including an amino acid sequence having a sufficient variable region sequence to impart specificity to an antigen and a constant region domain C_{L}, and fragments thereof.

In the present disclosure, the term "variable region or variable domain" refers to a part of an antibody molecule that exhibits many variations on the sequence while performing a function that specifically binds to an antigen, and in the variable region, there are the complementarity determining regions CDR1, CDR2 and CDR3. A framework region (FR) portion exists between the CDRs to support a CDR ring. The "complementarity determining region" is a ring-shaped region involved in antigen recognition, and the specificity of the antibody for the antigen is determined as the sequence of this region changes.

The term "single chain fragment variable (scFv)" used in the present disclosure refers to a single-chain antibody formed by expressing only a variable region of the antibody through genetic recombination, and is a single-chain form in which the VH and VL regions of the antibody are linked to each other by a short peptide chain. The term "scFv" is intended to include a scFv fragment including an antigen-binding fragment, unless otherwise specified or otherwise understood from the context. This is obvious to those skilled in the art.

In the present disclosure, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for the antibody binding to an antigen or epitope.

In the present disclosure, the term "specifically binding" or "specifically recognizing" has the same meaning as commonly known to those skilled in the art, and means that an antigen and an antibody specifically interact with each other to cause an immunological response.

As used in the present disclosure, the term "antigen-binding fragment" refers to a fragment of the entire structure of immunoglobulin, and refers to a portion of a polypeptide including a portion capable of binding with the antigen. For example, the antigen-binding fragment may be scFv, (scFv)2, scFv-Fc, Fab, Fab' or F(ab')2, but is not limited thereto. The Fab of the antigen binding fragments has a structure having variable regions of the light and heavy chains, a constant region of the light chain, and a first constant region C_{H1} of the heavy chain, and has one antigen-binding site. The Fab' is different from Fab by having a hinge region containing one or more cysteine residues in a C-terminal of the heavy chain C_{H1} domain. The F(ab')2 antibody is fabricated when the cysteine residues in the hinge region of Fab' form disulfide bonds. Fv is a minimal antibody fragment having only a heavy chain variable region and a light chain variable region, and a recombination technique for generating the Fv fragment is widely known in the art. The two-chain Fv has the heavy chain variable region and the light chain variable region linked via a noncovalent bond, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 any amino acids, and appropriate sequences are known in the art. The antigen binding fragments may be obtained using protease (for example, the whole antibody is restriction-cleaved with papain to obtain Fab, and cleaved with pepsin to obtain an F(ab')2 fragment), or may be fabricated through genetic recombination technology.

In the present disclosure, the term "hinge region" refers to a region included in the heavy chain of the antibody, and means a region which is present between the C_{H1} and C_{H2} regions and functions to provide flexibility of the antigen binding domain in the antibody. For example, the hinge may be derived from a human antibody, and specifically, derived from IgA, IgE, or IgG, such as IgG1, IgG2, IgG3, or IgG4.

The antibody or immunologically active fragment thereof of the present disclosure may be prepared by any method known in the art. After encoding a polypeptide sequence of the antibody or immunologically active fragment thereof of the present disclosure, if desired, the polypeptide sequence is cloned into a host cell and used to form a nucleic acid that is expressed and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

A nucleic acid encoding the antibody or immunologically active fragment thereof of the present disclosure may be inserted into an expression vector for protein expression. The expression vector generally includes a protein operably linked, i.e., functionally related to a control or regulatory sequence, a selectable marker, any fusion partner, and/or an additional element. Under appropriate conditions, the antibody or immunologically active fragment thereof according to the present disclosure may be produced by a method of inducing protein expression by culturing a host cell transformed with a nucleic acid, preferably an expression vector containing a nucleic acid encoding the antibody or immunologically active fragment thereof according to the present disclosure. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, the antibody or immunologically active fragment thereof according to the present disclosure is produced using E. coli, which has low production cost and high industrial utility value, as a host cell.

In one aspect, the present disclosure relates to a chimeric antigen receptor (CAR) including an antibody or immunologically active fragment thereof that binds to ITGB2 of the present disclosure as an antigen-binding domain.

In one aspect, the present disclosure relates to a recombinant vector including a gene encoding the chimeric antigen receptor.

In one aspect, the present disclosure relates to a chimeric antigen receptor expressing cell transformed with the recombinant vector.

In one embodiment, the chimeric antigen receptor expressing cell may be a chimeric antigen receptor expressing cell, a chimeric antigen receptor expressing macrophage (CAR-macrophage), a CAR expressing T (CAR-T) cell, a CAR expressing-gamma-delta T (CAR-Gamma-delta T) cell, or a CAR natural killer (CAR-NK) cell.

As used in the present disclosure, the term "chimeric antigen receptor (CAR)" refers to a non-naturally occurring receptor capable of imparting specificity for a specific antigen to an immune effector cell. Typically, the CAR refers to a receptor used to transplant the specificity of a monoclonal antibody into a T cell. The CAR usually consists of an ectodomain, a transmembrane domain, and an ectodomain.

The ectodomain includes an antigen recognition region, and the transmembrane domain of the CAR is in a form linked to the ectodomain, and may be derived naturally or synthetically. In the case of being derived naturally, the transmembrane domain may be derived from a membrane-binding or membrane-permeating protein, and may be a portion derived from a transmembrane domain of various proteins, such as an alpha, beta or zeta chain of the T cell receptor, CD28, CD3 epsilon, CD45, CD4, CD5, CDS, CD9, CD16, CD22, CD33, CD37, CD64, CD80, CD86, CD134, CD137, CD 154 or CD8. The sequence of such a transmembrane domain may be obtained from documents known in the art that disclose the membrane-permeating region of the membrane-permeating protein, but is not limited thereto.

In addition, when the transmembrane domain is synthesized, the transmembrane domain may mainly include hydrophobic amino acid residues such as leucine and valine, and examples thereof may be present in a transmembrane domain in which a triplet of phenylalanine, tryptophan, and valine is synthesized, but are not limited thereto. Sequence information for such a transmembrane domain may be obtained from documents known in the art for the synthesized transmembrane domain, but is not limited thereto.

In the CAR of the present disclosure, the endodomain is a part of the domain of the CAR existing within a cell, and a form linked with the transmembrane domain. The endodomain of the present disclosure may include an intracellular signaling domain characterized by causing T cell activation, preferably T cell proliferation, when an antigen binds to an antigen binding site of the CAR. The intracellular signaling domain is not particularly limited in type as long as the intracellular signaling domain is a part that transmits a signal that may lead to T cell activation when an antibody binds to an antigen binding site existing outside the cell, and may be used with various types of intracellular signaling domains. For example, the intracellular signaling domain may be an immunoreceptor tyrosine-based activation motif or ITAM, and the ITAM may be derived from CD3 zeta (ξ), FcR gamma, FcR beta, CD3 gamma, CD3 delta, CD3 epsilon, CDS, CD22, CD79a, CD79b, CD66d or FcεRIγ, but is not limited thereto.

The CAR includes a single-chain fragment variable region (scFv) of an antibody specific for a tumor-associated antigen (TAA) that is coupled to the cytoplasmic domain of a T-cell signaling molecule via a hinge and a transmembrane region. Most conventional lymphocyte activating moieties include T-cell costimulatory (e.g., CD28, CD137, OX40, ICOS, and CD27) domains in a tandem with a T-cell triggering (e.g., CD3ζ) moiety. CAR-mediated adoptive immunotherapy allows a CAR-grafted cell to directly recognize a TAA on a target tumor cell in a non-HLA-restricted manner.

As used in the present disclosure, the term "chimeric antigen receptor expressing T (CAR-T) cell" means a T cell expressing a CAR. The CAR-T cell i) recognizes a cancer antigen in a manner independent of human leukocyte antigen (HLA) to have the advantage of being able to treat cancers that evade the action of anticancer agents by reducing HLA expression on the cell surface, ii) is independent of HLA type to be used for treatment regardless of the patient's HLA type, and iii) has the advantage of being able to produce a large number of cancer-specific T cells in a short period of time to exhibit excellent anticancer effects.

The T cell includes CD4⁺ T cells (helper T cells, TH cells), CD8⁺ T cells (cytotoxic T cells, CTLs), memory T cells, regulatory T cells (Treg cells), natural killer T cells, etc., and the T cells into which the CAR is introduced in the present disclosure are preferably CD8⁺ T cells, but are not limited thereto.

In one embodiment, the present disclosure relates to a T-cell engager including an antibody or immunologically active fragment thereof that binds to ITGB2.

In one embodiment, the T cell engager may be, for example, a bispecific T-cell engager (BiTE). The BiTE is a class of artificial bispecific monoclonal antibodies, which is a fusion protein consisting of amino acid sequences from four different genes or two single-chain variable region fragments (scFvs) of different antibodies on a single peptide chain of about 55 kilodaltons. One of the scFvs binds to a T cell via the CD3 receptor, and the other binds to a tumor cell via a tumor-specific molecule. Similarly to other bispecific antibodies, and unlike common monoclonal antibodies, the BiTE forms a link between a T cell and a tumor cell. The BiTE allows T cells to exhibit cytotoxic activity on tumor cells independently of the presence of MHC I or costimulatory molecules by producing proteins such as perforin and granzymes. These proteins are introduced into tumor cells and initiate apoptosis.

In one aspect, the present disclosure relates to a pharmaceutical composition for preventing or treating cancer, including a drug delivery conjugate, a peptide-drug conjugate or an antibody-drug conjugate of the present disclosure as an active ingredient.

In one embodiment, the present disclosure relates to a cancer immunotherapy agent including a drug delivery conjugate, a peptide-drug conjugate or an antibodydrug conjugate of the present disclosure as an active ingredient.

In one embodiment, the cancer may be an M2 tumor-associated macrophage-mediated cancer.

In one embodiment, the composition may be administered to a patient in whom the expression of ITGB2 is upregulated in M2 macrophages, compared to M0 macrophages and M1 macrophages.

In one embodiment, the cancer may be any one selected from the group consisting of brain tumor, melanoma, myeloma, non-small cell lung cancer, oral cancer, liver cancer, stomach cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma, and may be an anticancer agent-resistant cancer.

The pharmaceutical composition of the present disclosure may be used as a single therapy, but may also be used in combination with other conventional biological therapy, chemotherapy or radiation therapy, and may treat more effectively cancer in the case of such concurrent therapy. In the case of using the present disclosure for preventing or treating cancer, a chemotherapeutic agent that may be used with the composition includes cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like. Radiation therapy that may be used together with the composition of the present disclosure includes X-ray irradiation and γ-ray irradiation.

As used in the present disclosure, the term "prevention" means all actions that inhibit or delay the occurrence, spread, and recurrence of cancer by administration of the composition according to the present disclosure.

The therapeutically effective amount of the composition of the present disclosure may vary depending on many factors, for example, an administration method, a target site, a condition of a patient, and the like. Accordingly, when used in the human body, a dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. In the present disclosure, the "pharmaceutically effective dose" refers to an amount enough to treat the disease at a reasonable benefit/risk ratio applicable to medical treatment and not to cause side effects. An effective dose level may be determined according to factors including the patient's health status, the type and severity of a disease, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, a diluent, an excipient, or a combination of two or more thereof, which are commonly used in biological agents. As used in the present disclosure, the term "pharmaceutically acceptable" refers to exhibiting non-toxic properties to normal cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may further contain one or more active ingredients exhibiting the same or similar functions. The composition of the present disclosure includes 0.0001 to 10 wt%, preferably 0.001 to 1 wt% of the protein, based on the total weight of the composition.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 part by weight to 90 parts by weight based on the composition, but is not limited thereto.

The composition of the present disclosure may be administered parenterally (e.g., applied intravenously, subcutaneously, intraperitoneally or topically) or orally according to a desired method, and the range of the dose may vary depending on the weight, age, sex, and health condition of a patient, a diet, an administration time, an administration method, an excretion rate, the severity of a disease, etc. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure relates to a method for screening a substance that specifically delivers a drug to M2 tumor-associated macrophages, including treating ITGB2 (Integrin beta 2) with a candidate substance; and selecting the candidate substance that binds to ITGB2.

In one embodiment, the method may select a candidate substance that specifically binds to CD18 in an extended active conformation.

In one aspect, the present disclosure relates to a method for selecting a patient having responsiveness to an anticancer agent targeting M2 macrophages, including determining the expression level of ITGB2 in an isolated sample.

In one embodiment, the method may further include determining a patient as having responsiveness to an anticancer agent targeting M2 macrophages, when the expression level of ITGB2 in M2 macrophages is higher than that in M0 macrophages and M1 macrophages.

In one aspect, the present disclosure relates to a solid cancer diagnostic marker including an ITGB2 gene or a protein expressed from the gene.

In one aspect, the present disclosure relates to a composition for diagnosing solid cancer, including an agent for measuring the expression level of ITGB2 at an mRNA or protein level.

In the one embodiment, the agent for measuring the expression level at the mRNA level may be a primer pair, a probe, or a primer pair and a probe that specifically recognize a nucleic acid sequence of the gene, a nucleic acid sequence complementary to the nucleic acid sequence, and fragments of the nucleic acid sequence and the complementary sequence, and the measurement may be performed by one or more methods selected from the group consisting of polymerase chain reaction, real-time RT-PCR, reverse transcription PCR, competitive RT-PCR, nuclease protection assay (RNase, S1 nuclease assay), in situ hybridization, nucleic acid microarray, northern blot, DNA chips, multiplex PCR, or ddPCR.

In one embodiment, the agent for measuring the expression level at the protein level may be antibodies, antibody fragments, aptamers, avidity multimers or peptidomimetics that specifically recognize the full-length protein or fragments thereof of the gene, and the measurement may be performed by one or more methods selected from the group consisting of western blot, enzyme linked immunosorbent assay (ELISA), radioimmunoassay (RIA), radioimmunodiffusion, immunoelectrophoresis, tissue immunostaining, immunoprecipitation assay, complement fixation assay, FACS, mass spectrometry or protein microarray.

As used in the present disclosure, the term "detection" or "measurement" refers to quantifying the concentration of an object to be detected or measured.

As used in the present disclosure, the term "primer" refers to a nucleic acid sequence having a short free 3 hydroxyl group, and a short nucleic acid sequence capable of forming a base pair with a complementary template and serving as a starting point for copying a template strand. The primer may initiate DNA synthesis in the presence of a reagent for polymerization (i.e., DNA polymerase or reverse transcriptase) and four different nucleoside triphosphates in appropriate buffer and temperature.

As used in the present disclosure, the term "probe" refers to a nucleic acid fragment such as RNA or DNA corresponding to several bases to several hundred bases in length capable of forming specific binding to mRNA, and is labeled to identify the presence or absence of specific mRNA. The probe may be prepared in the form of an oligonucleotide probe, a single stranded DNA probe, a double stranded DNA probe, an RNA probe, or the like. In the present disclosure, hybridization is performed using a probe complementary to the ITGB2, and the gene expression level may be diagnosed through hybridization. Selection of an appropriate probe and hybridization conditions may be modified based on those known in the art, and thus, the present disclosure is not particularly limited thereto.

The primer or probe of the present disclosure may be chemically synthesized using a phosphoramidite solid support method, or other well-known methods. Such a nucleic acid sequence may also be modified using many means known in the art. Non-limiting examples of such a modification include methylation, encapsulation, substitution with one or more homologs of natural nucleotides, and modifications between nucleotides, for example, modification to uncharged linkers (e.g., methyl phosphonate, phosphotriester, phosphoroamidate, carbamate, etc.) or charged linkers (e.g., phosphorothioate, phosphorodithioate, etc.).

In the present disclosure, suitable conditions for hybridizing the probe with a cDNA molecule may be determined in a series of processes by an optimization procedure. This procedure is performed as a series of processes by those skilled in the art to establish a protocol for use in a laboratory. For example, conditions such as temperature, concentration of components, hybridization and washing time, buffer components, and pH and ionic intensities thereof depend on various factors such as a length and a GC amount of the probe, a target nucleotide sequence, and the like. Detailed conditions for hybridization may be identified in Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999). For example, under the stringent conditions, a high stringent condition means hybridization at a condition of 65°C in 0.5 M NaHPO₄, 7% sodium dodecyl sulfate (SDS), and 1 mM EDTA, and washing at a condition of 68°C in 0.1× Standard saline citrate (SSC)/0.1% SDS. Alternatively, the high stringent condition means washing at a condition of 48°C in 6x SSC/0.05% sodium pyrophosphate. A low stringent condition means washing at a condition of 42°C in 0.2× SSC/0.1% SDS.

In one aspect, the present disclosure relates to a method for predicting responsiveness or diagnosing prognosis for a cancer immunotherapy agent targeting M2 macrophages, including confirming the expression level of ITGB2 in a sample treated with the cancer immunotherapy agent of the present disclosure.

In one embodiment, the method may further include determining that the responsiveness to the cancer immunotherapy agent is good when the expression level of ITGB2 protein in the sample is reduced after treatment with the cancer immunotherapy agent.

In one embodiment, the sample may be blood, serum, plasma, amniotic fluid, saliva, ascites, bone marrow, tears, bile, lung lavage fluid, cerebrospinal fluid, pleural effusion, synovial fluid, lymph, semen, urine, or a solution obtained by extracting proteins from a tissue biopsy or cells.

In one aspect, the present disclosure relates to a method for providing information necessary for diagnosing cancer, including confirming the expression level of ITGB2 in a sample isolated from a subject; comparing the expression level of ITGB2 with that of a control group; and classifying a subject in which ITGB2 is overexpressed compared to the control group.

In one embodiment, the method may further include determining that a subject in whom ITGB2 is overexpressed compared to the control group is more likely to have cancer.

In one aspect, the present disclosure relates to a method for treating cancer including administering to a subject suffering from cancer a pharmaceutically effective amount of the drug delivery conjugate of the present disclosure.

In one embodiment, the subject may be a patient in whom the expression of ITGB2 is upregulated in M2 macrophages, compared to M0 macrophages and M1 macrophages.

In one aspect, the present disclosure relates to a use of the drug delivery conjugate of the present disclosure for the preparation of a pharmaceutical composition for the prevention and treatment of cancer.

Hereinafter, the present disclosure will be described in more detail with reference to the following Examples. However, the following Examples are only intended to embody the contents of the present disclosure, and the present disclosure is not limited thereto.

### [Modes of the Invention]

### Example 1. Synthesis of peptides targeting M2 tumor-associated macrophages (M2 TAMs)

Melittin and TAMpep826 in Table 1 below were synthesized and supplied by GenScript (Piscataway, NJ, USA), and dissolved in D-PBS at 5 mg/ml for use in subsequent experiments.

**[Table 1]**

| Name | Sequence | SEQ ID NO: | Description |
|---|---|---|---|
| Melittin | GIGAVLKVLTTGLPAL ISWIKRKRQQ | 1 | Melittin peptide |
| TAMpep8 26 | VLTTGLPALISWIKRK RQQ | 2 | Peptide in which first 7 amino acids of melittin are removed |

### Example 2. M2 TAM targeting analysis

### 2-1. Confirmation of affinity of melittin to M2 TAM

To confirm whether the melittin fabricated in Example 1 specifically bound to M2 tumor-associated macrophages (M2 TAMs), a human monocyte cell line THP-1 was differentiated into M0, M1, and M2 macrophages, and treated with FITC-conjugated melittin, and then confirmed by flow cytometry. Specifically, THP-1 cells were treated with 100 nM phorbol 12-myristate 13-acetate (PMA) at 37°C for 24 hours (M0 macrophages), and the differentiated M0 macrophages were incubated with 100 nM LPS and 20 ng/ml IFN-γ at 37°C for 72 hours to be differentiated into M1 macrophages (M1), and incubated with 20 ng/ml IL-4 and IL-13 at 37°C for 72 hours to be differentiated into M2 macrophages (M2). The differentiated cells were treated with 50 nM FITC-conjugated melittin for 1 hour, detected with BD FACS CantoII instruments (BD biosciences, San Jose, CA, USA), and analyzed with FlowJo software (BD biosciences).

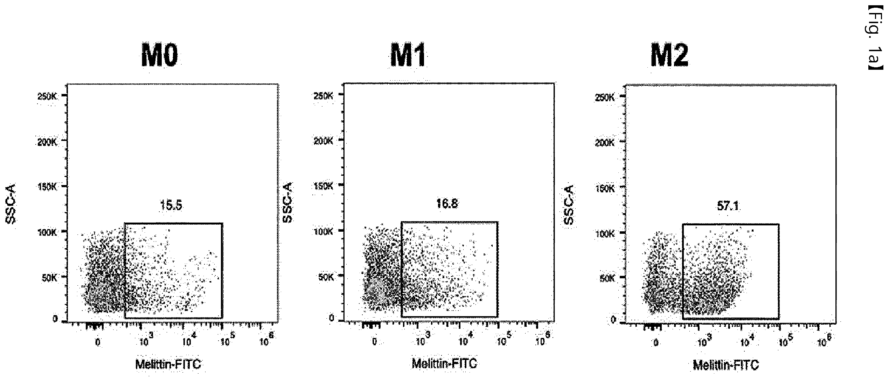

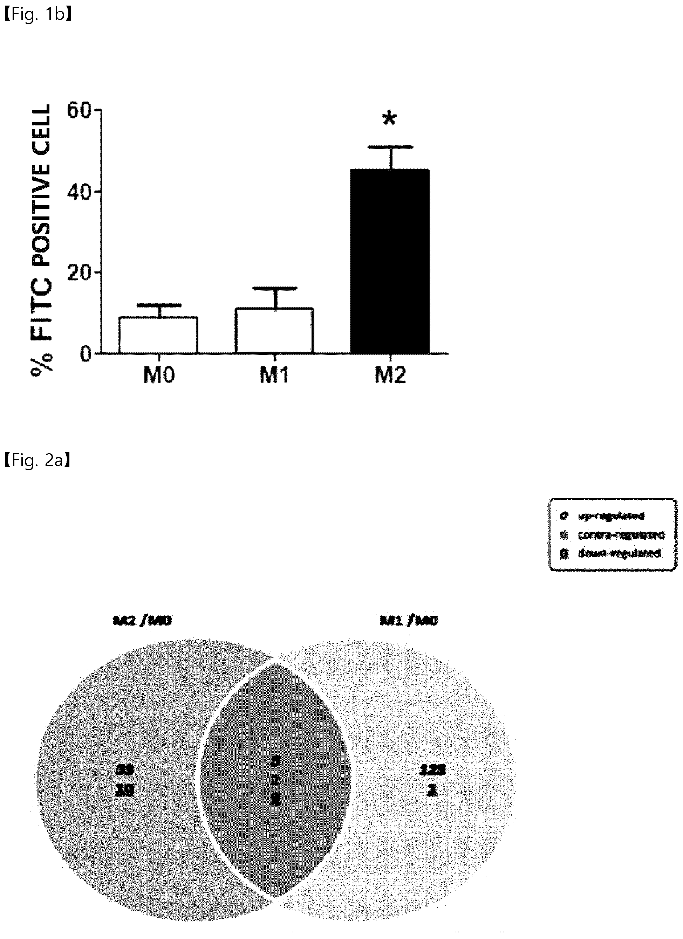

As a result, FITC-positive cells were significantly increased in M2 macrophages compared to M0 and M2 macrophages (FIGS. 1A and 1B). Through this, it was confirmed that melittin preferentially bound to M2 macrophages.

### 2-2. Identification of M2 TAM binding target protein of melittin

To determine which protein of M2 macrophages was involved in the specific binding of melittin to M2 macrophages, biotin-conjugated melittin (melittin-biotin) was synthesized. THP-1 cells (5 × 10⁶ cells) were differentiated into M0, M1, and M2 macrophages, and then collected using a scraper, washed by centrifugation (300 X g, 5 min), and cell membrane proteins of macrophages were obtained using a cell membrane protein extraction kit (Thermo Fisher scientific). The proteins reacted with melittin-biotin (200 µg) in which biotin bound to melittin at room temperature (20 to 24°C) for 1 hour, and then the proteins bound to melittin were obtained using a streptavidin resin (Thermo Fisher scientific). To remove the salt, 100 µl each of 100% methanol, 0.1% formic acid, and 80% CAN were added to an 18 Micro Spin-Column, and the sample was loaded onto the prepared column, and added with 50 µl of 0.1% formic acid to remove unnecessary substances. Thereafter, 100 µl of 80% CAN was added to elute the peptides. After desalting, the solution was completely removed by drying with a Speed-vac, and analyzed by LC-MS/MS using a UPLC-Exactive equipment (Thermo Fisher Scientific) under the conditions shown in Table 2 below. Data were analyzed for proteins upregulated in M1 and M2 macrophages compared to M0 macrophages using MaxQuant and Mass Profiler Professional (MPP). For data analysis, LC-MS/MS data for each sample were analyzed using Proteome Discoverer, and performed by LFQ (Label-Free Quantification) using a human database of Uniprot as the database. Modifications included Oxidation (M), Carbamyl (N-term), and Carbamidomethyl (C), and the Baseline option was set to none.

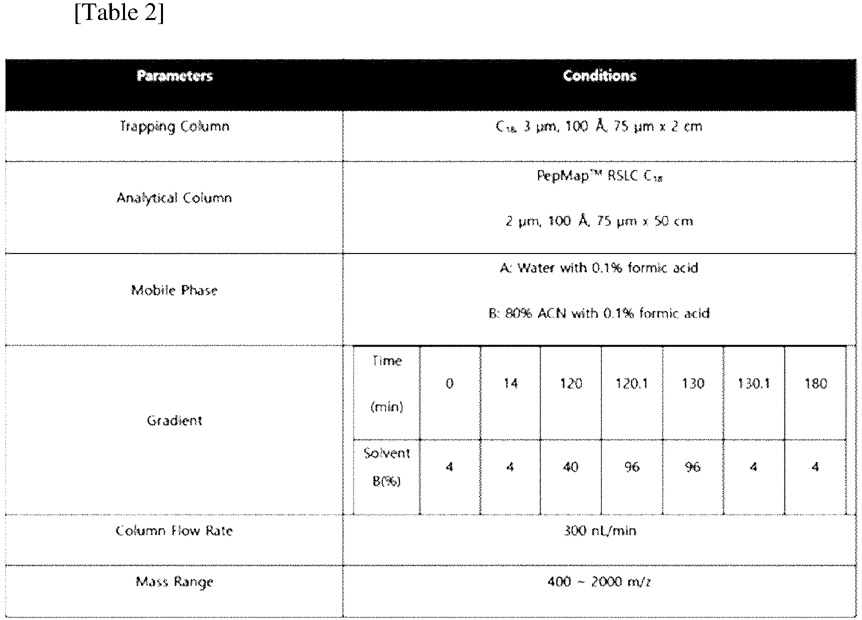

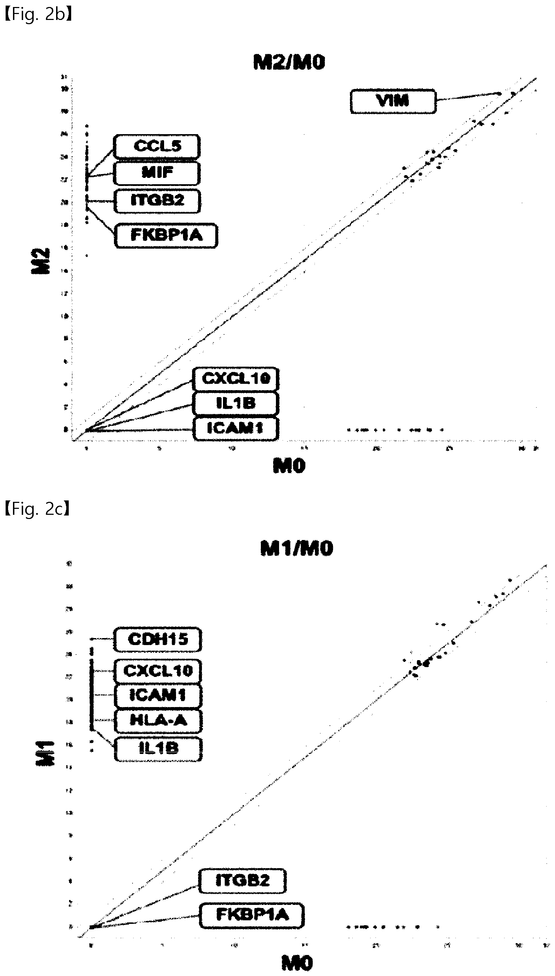

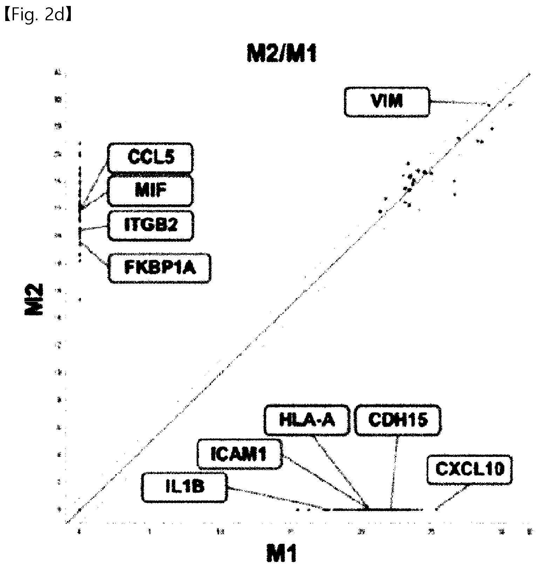

As a result, 53 proteins were upregulated in M2/M0 and 123 proteins were upregulated in M1/M0 (FIG. 2A). More upregulated proteins interacted with M1 macrophages than with M2 macrophages, but proteins present in the cell membrane did not interact. Among the membrane proteins, ITGB2 was found to be upregulated in M2 macrophages (FIG. 2B). In addition, cytokines such as IL-10 and CXCL10, known as markers of M1 macrophages, were upregulated in M1 macrophages (FIG. 2V). In addition, proteins were identified to be upregulated in M2 compared to M1, but among the membrane proteins, only ITGB2 was identified to be upregulated in M2 macrophages (FIG. 2D). Through this, it was confirmed that melittin interacted and bound with the membrane protein ITGB2 of M2 macrophages.

### 2-3. Identification of M2 TAM binding target protein of TAMpep826

As in Example 2-2 above, TAMpep826-biotin reacted with cell membrane proteins extracted from M0, M1, and M2 macrophages to identify target proteins through LC-MS/MS proteomics.

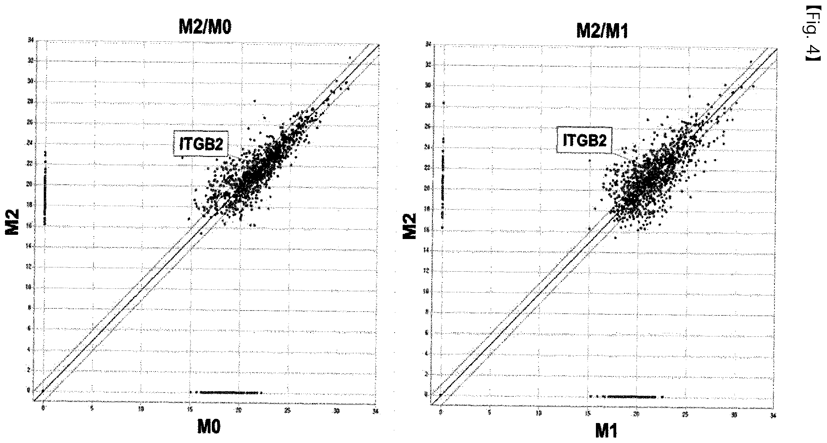

As a result, ITGB2, among the cell membrane proteins, was found to be expressed at a higher level in M2 macrophages than in M0 and M1 macrophages (FIG. 4), and was selected as a M2 macrophage binding target protein of TAMpep826.

### Example 3. Confirmation of ITGB2 expression in M2 macrophages

### 3-1. Confirmation of ITGB2 mRNA expression

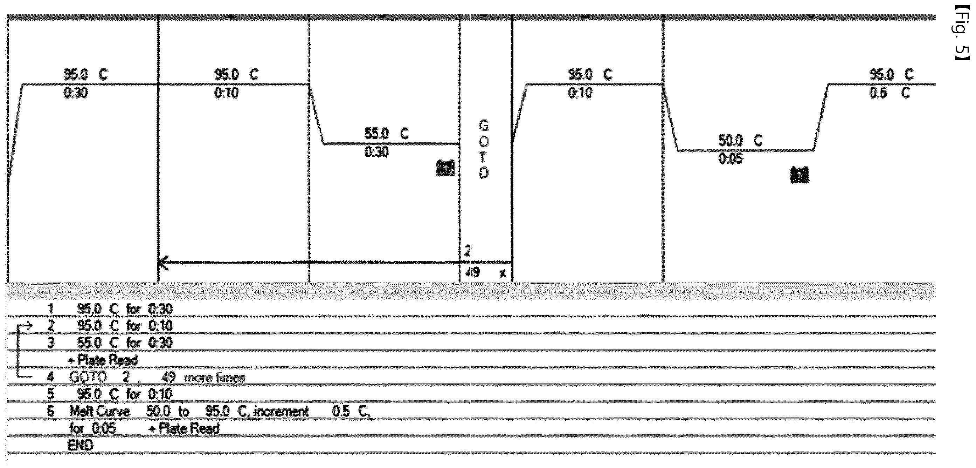

Real-time polymerase chain reaction was performed to evaluate the mRNA expression levels of ITGB2 in M0, M1, and M2 macrophages and TAMs. Specifically, RNA was extracted from cells differentiated into M0, M1, or M2 macrophages using an easy-BLUE total RNA Extraction Kit (iNtRON), and cDNA was synthesized using CycleScript-Reverse Transcriptase (Bioneer). Primers for an Actin or ITGB2 gene (Table 3) were used and real-time PCR (CFX96, Bio-rad) was performed under the conditions of FIG. 5 to compare and analyze the expression level of each gene relative to Actin through relative quantification.

**[Table 3]**

| Target cDNA | Forward/Reverse | Sequences (5' to 3') |
|---|---|---|
| β-Actin | F | GTGCTATGTTGCTCTAGACTTCG |
| | R | ATGCCACAGGATTCCATACC |
| ITGB2 | F | GAGGTCAGGAGGATTTGGGG |
| | R | TACAAGTGTTGGGGAGCCAG |

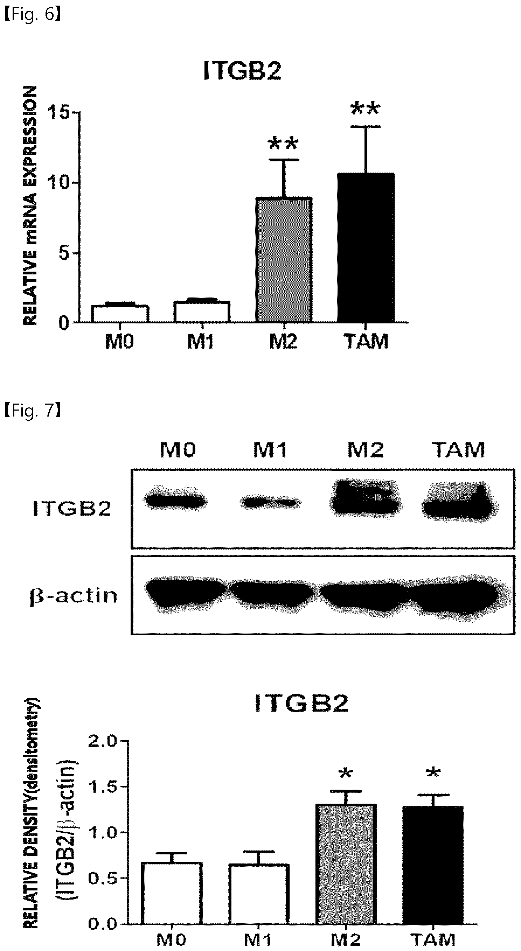

As a result, mRNA expression of ITGB2 was significantly increased in M2 macrophages and TAMs compared to M0 and M1 (FIG. 6).

### 3-2. Confirmation of ITGB2 protein expression

Western blot analysis was performed to evaluate the protein expression levels of ITGB2 in M0, M1, and M2 macrophages and TAMs. Specifically, proteins were extracted from each cell differentiated into M0, M1, and M2 macrophages using a Proprep solution (iNtRON), and the proteins were transferred to the membrane by electrophoresis. The membrane was blocked in 5% BSA, treated with a primary antibody (ITGB2) for 24 hours at 4°C, treated with a secondary antibody for 1 hour at room temperature, and then treated with the D-Plus^{™} ECL Pico System (Dong-In LS), and protein bands were photographed using the Davinch-Chemi^{™} imaging system (Intoxia).

As a result, it was shown that the protein expression of ITGB2 was significantly increased in M2 macrophages and TAMs compared to M0 and M1 (FIG. 7).

### 3-3. Confirmation of ITGB2 protein expression by immunofluorescence

Immunofluorescence analysis was performed to evaluate the protein expression levels of ITGB2 in M0, M1, and M2 macrophages and TAMs. Specifically, each cell differentiated into M0, M1, and M2 macrophages was fixed with 4% formalin, washed with PBS, blocked in 5% BSA, and then treated with a primary antibody (ITGB2) for 24 hours at 4°C and a secondary antibody for 2 hours at room temperature and stained. After DAPI mounting, the stained cells were photographed with fluorescence using LSM800 (Zeiss).

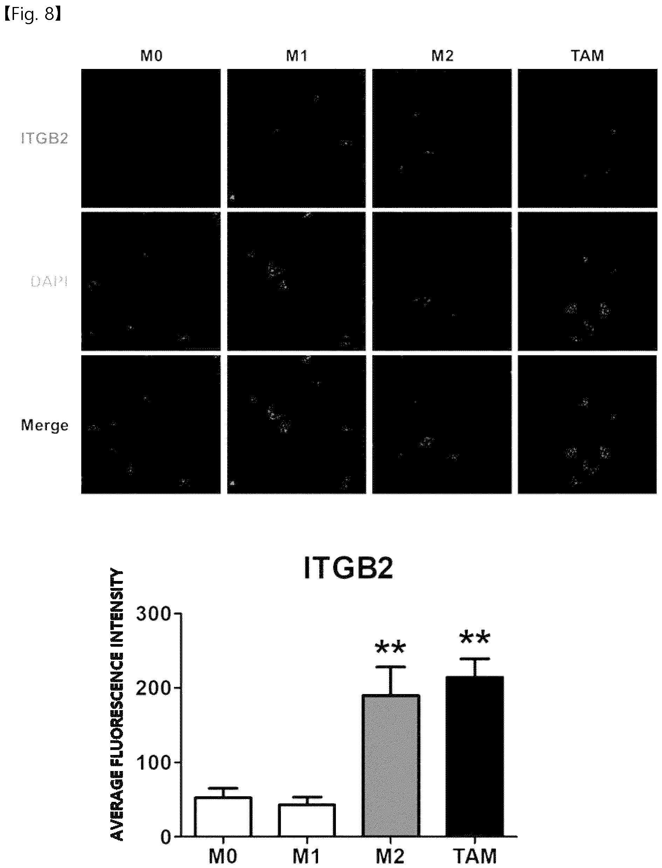

As a result, the protein of ITGB2 showed higher expression levels in M2 macrophages and TAMs compared to M0 and M1 (FIG. 8).

### Example 4. Confirmation of interaction between M2 TAM targeting peptide and ITGB2

### 4-1. Confirmation of ITGB2 expression on M2 macrophage cell membrane

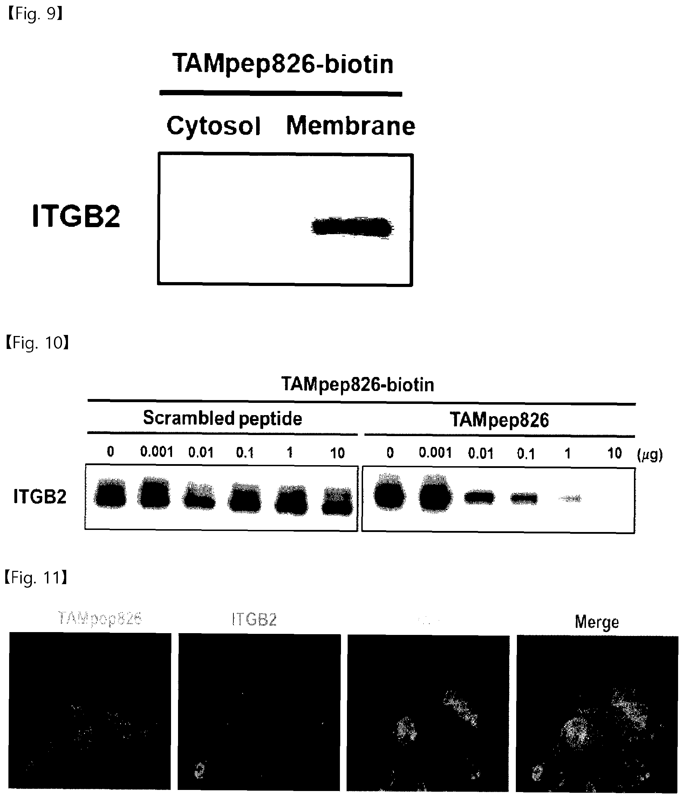

To determine whether ITGB2 was expressed on the cell membrane of M2 macrophages, membrane proteins and cytosol proteins were isolated and extracted from M2 macrophages, respectively, and reacted with biotinylated TAMpep826 to evaluate the expression of ITGB2 by Western blot analysis. As a result, it was shown that the ITGB2 protein was expressed on the cell membrane of M2 macrophages (FIG. 9).

### 4-2. Confirmation of interaction between TAMpep826 and ITGB2

To determine whether TAMpep826 interacted with ITGB2 in M2 macrophages, the expression of ITGB2 was evaluated through competitive reaction of TAMpep826 and biotinylated TAMpep826. Specifically, cells differentiated into M2 macrophages were collected with a scraper, the cells were washed by centrifugation (300 X g, 5 min), and the cell membrane proteins of macrophages were obtained using a cell membrane protein extraction kit (Thermo Fisher scientific). After pretreatment with TAMpep826 or scramble peptide for each concentration (0.001, 0.01, 0.1, 1, and 10 µg) for 1 hour, the proteins reacted with biotin-conjugated TAMpep826 (200 µg) at room temperature (20 to 24°C) for 1 hour, and then the proteins bound to TAMpep826 were obtained using Dynabeads^{™} M-280 Streptavidin (Thermo Fisher scientific). The expression of ITGB2 was confirmed by Western blot analysis of the proteins.

As a result, when pretreated with the scrambled peptide, the expression of ITGB2 bound to biotinylated TAMpep826 was not affected, whereas when pretreated with TAMpep826, ITGB2 expression was suppressed depending on a concentration (FIG. 10). Through this, it may be confirmed that TAMpep826 interacts with ITGB2 on the cell membrane of M2 macrophages.

### 4-3. Confirmation of interaction between TAMpep826 and ITGB2 using immunofluorescence

To determine whether ITGB2 and TAMpep826 were co-localized on the cell membrane of M2 macrophages, immunofluorescence analysis was performed using FITC-conjugated TAMpep826. As a result, it was shown that TAMpep826 and ITGB2 were intensively distributed in the cell membrane of M2 macrophages (FIG. 11).

### 4-4. Identification of binding sites of TB511 and ITGB2

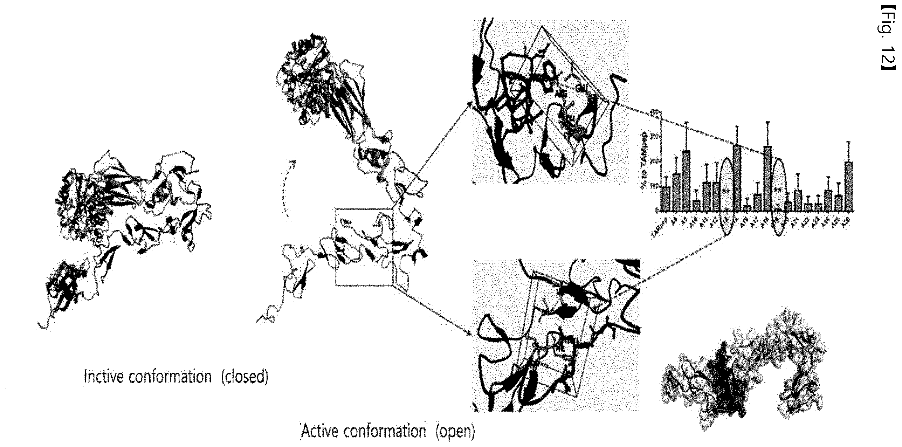

Based on a sequence of a cysteine rich region of CD18 (ITGB2) (uniport_p05107, Cystein-rich tandem repeats region: 449-617), a tertiary structure was constructed using a trRosetta algorithm (https://yanglab.nankai.edu.cn/trRosetta/). Thereafter, a docking simulation of TB511 and CD18 was performed using a CD18 PDB file created by trRosetta and an amino acid sequence of TB511 via a CABS-dock server (http://biocomp.chem.uw.edu.pl/CABSdock/). Among 10 models generated from the docking simulation result, a representative model was selected based on an alanine substitution result of the TB511 sequence (FIG. 12).

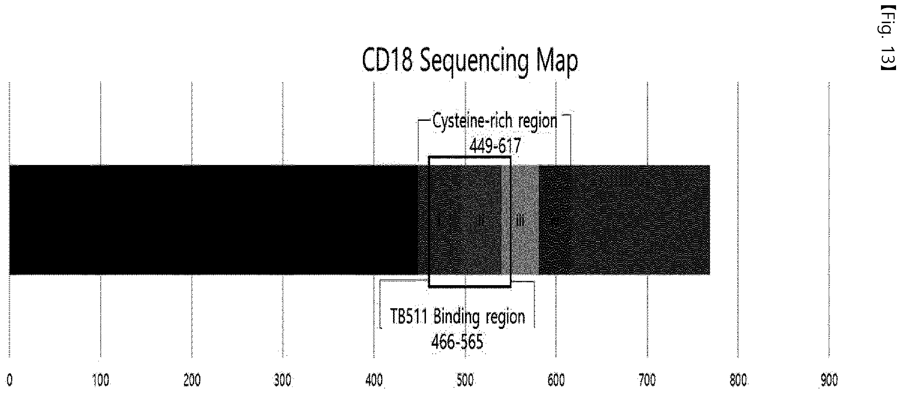

As a result, it was predicted that LEU(6) of TB511 bound to LEU(528), THR(538), LEU(556), CYS(557) and PHE(558) sites of CD18, and TRP(12) of TB511 bound to GLU(466), ILE(469), CYS(470) and ARG(471) of CD18. In addition, it was shown that when LEU(6) and TRP(12) of TB511 were each substituted with alanine to simulate binding to CD18, both the two regions did not bind to any sequence of CD18. Accordingly, LEU(6) and TRP(12) of TB511 are key parts in the docking between TB511 and CD18, and TB511 may be predicted to bind to positions 466 to 565 of CD18 (FIG. 13).

### Example 5. Synthesis of ITGB2 binding moieties

### 5-1. Synthesis of ITGB2-binding peptide-drug conjugate (PDC)

Melittin-dKLA and TB511 peptides in Table 4 below were synthesized and supplied by GenScript (Piscataway, NJ, USA) and were dissolved in D-PBS at 5 mg/ml and used in subsequent experiments. dKLA was linked through amide bonds between peptides, and a linker consisting of four glycines and one serine was placed in the middle to separate two ends so as to minimize the interaction and folding between melittin or TAMpep826 and dKLA. In addition, a D-enantiomer other than an L-enantiomer of KLA was used to minimize degradation in the body. In addition, in order to fabricate M-DM1, maleimide-modified melittin (GenScript, Beijing, China) (100 µM, 1 mL, 0.1 mmol) dissolved in 25 mM of a sodium borate buffer (25 mM NaCl, 1 mM EDTA, pH 8.0) was added with DM1 (Mertansine) (MedChem Express, Princeton) (1.1 equivalents, 10 mM in DMF) and dimethylformamide (DMF), and reacted at 37°C for 1 hour. Then, the product was filtered three times by ultrafiltration in Dulbecco's phosphate-buffered saline (DPBS; Welgene), and thus DM1 bound to the N-terminus of maleimide-modified melittin M to fabricate PDC, which was named as M-DM1. 5 µL of the fabricated M-DM1 was injected by reverse-phase high-performance liquid chromatography (HPLC) using a Poroshell 120 C18 column (2.7 µm, 3 × 50 mm, Agilent, Santa Clara, CA, USA) at a flow rate of 0.5 mL/min, and then the characteristics were analyzed.

**[Table 4]**

| Name | Sequence | SEQ ID NO: | Description |
|---|---|---|---|
| Melittin | GIGAVLKVLTTGLPALIS WIKRKRQQ | 1 | Melittin peptide |
| TAMpep8 26 | VLTTGLPALISWIKRKR QQ | 2 | Peptide in which first 7 amino acids of melittin are removed |
| Linker | GGGGS | 3 | |
| dKLA | d[KLAKLAKKLAKLAK] | 4 | Pro-apoptosis peptide |
| Melittin-dKLA | GIGAVLKVLTTGLPALIS WIKRKRQQ-GGGGS- | 5 | Peptide with linker and drug dKLA attached to melittin |
| | d[KLAKLAKKLAKLAK] | | |
| TB511 | VLTTGLPALISWIKRKR QQ-GGGGS- d[KLAKLAKKLAKLAK] | 6 | Peptide with linker and drug dKLA attached to TAMpep826 |
| M-DM1 | | | PDC conjugated with drug DM1 to maleimide-modified melittin |

### 5-2. Synthesis of ITGB2-binding antibody-drug conjugate (ADC)

### 5-2-1. ADC fabrication of anti-CD18 antibody and DM1

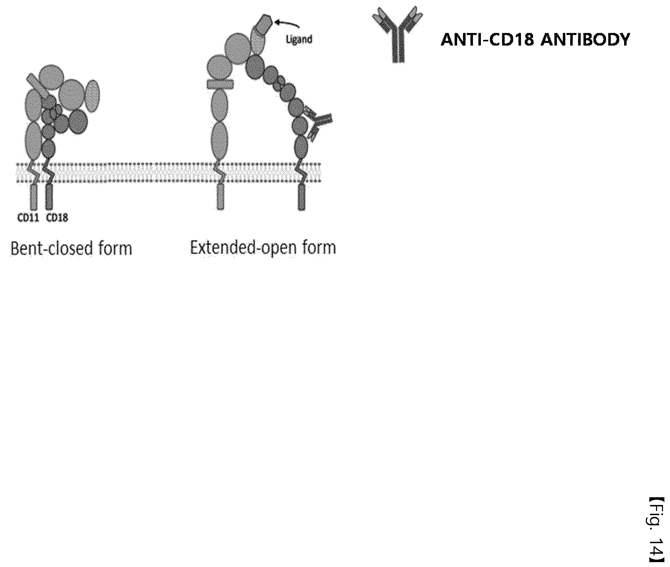

To fabricate an antibody that specifically bound to an active conformation of CD18, one of the macrophage-1 antigens (Mac-1), in M2 TAM (FIG. 14), a culture medium of hybridoma (MM18/2.a.8) cells secreting anti-CD18 antibodies was collected, centrifuged at 2,500 rpm and 4°C for 10 minutes to obtain a supernatant, and the supernatant was purified using protein G-Sepharose column chromatography. The antibody solution slowly passed through a protein G-Sepharose column (Pharmacia, Sweden) pre-equilibrated with PBS, and the column was connected to a peristatic pump and washed sufficiently with PBS. After washing, the antibody was eluted with 0.2 M glycine-HCl (pH 2.7). At this time, the eluate was buffered in a tube containing 1 M Tris (pH 9.0) prepared in advance. This antibody was used after dialysis in PBS. The amount of purified antibody was measured using a BCA^{™} protein assay kit (Pierce), and the avidity of the antibody was confirmed through flow cytometry. The purified anti-CD18 antibody was buffer-replaced with PBS (pH 7.4), and an anti-CD18 antibody (1 mg/ml, 6.67 µM) was mixed with SMCC-DM1 (mertansine, 120 µM) (MedChemExpress) dissolved in DMSO at a ratio of 1 : 18, and reacted at room temperature for 4 to 20 hours. To remove unreacted DM1, CD18-DM1 was buffer-replaced with PBS (pH 7.4) using a 10 KDa cutoff centrifugal filter, and filtered through a 0.2 µm syringe filter. The amount of DM1 (252 nm) bound to anti-CD18 antibody (280 nm) was measured using UV-Vis spectroscopy, and a drug antibody ratio (DAR) of DM1 to the anti-CD18 antibody was calculated, and the DAR value was 5.8. The fabricated ADC in which the anti-CD18 antibody and DM1 were bound was named as CD18-DM1.

### 5-2-2. ADC fabrication of anti-CD18 antibody and monomethyl auristatin E (MMAE)

In Example above, the purified anti-CD18 antibody was buffer-replaced with PBS (pH 7.4), and in order to partially reduce the disulfide bonds of the CD18 antibody, 1 mM DTT (66.7 µM) was mixed with an anti-CD18 antibody (1 mg/ml, 6.67 µM) at a ratio of 1 : 10 and reacted at 37°C for 1 to 2 hours, and then buffer-replaced with PBS (pH 7.4). The partially reduced anti-CD18 antibody (1 mg/ml, 6.67 µM) was mixed with 10 mM Suo-Val-Cit-PAB-MMAE (MedChemExpress) at a ratio of 1 : 20 to 1 : 40, reacted at 4°C for 12 hours, and buffer-replaced with PBS (pH 7.4). The amount of ve-MMAE (248 nm) bound to an anti-CD18 antibody (280 nm) was measured using UV-Vis spectroscopy, and a drug antibody ratio (DAR) of vc-MMAE to the anti-CD18 antibody was calculated, and the DAR value was 2.8 to 5.4. The ADC fabricated by binding the anti-CD18 antibody and MMAE was named as CD18-MMAE.

### Example 6. Confirmation of M2 TAM binding to ITGB2 binding PDC

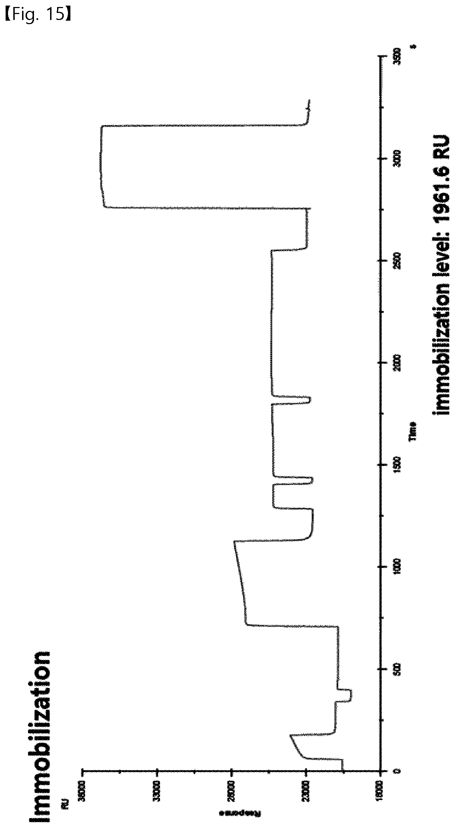

To confirm the binding affinity of ITGB2 and Melittin-dKLA, an ITGB2 protein was coated on a gold thin film sensor chip, and then ITGB2 antibody and Melittin-dKLA as positive controls were run, respectively, and the reflected light was measured and surface plasmon resonance (SPR) analysis was performed. Specifically, after ITGB2 was fixed on the sensor chip surface, immobilization was performed using amine coupling under conditions shown in Table 5 below (FIG. 15). Thereafter, various concentrations of ITGB2 antibody and Melittin-dKLA were run through the ITGB2-coated sensor chip to observe association and dissociation sections, and an intermolecular association rate constant (kinetic parameter) was calculated using a sensorgram under conditions of the intermolecular association rate constant analysis (kinetic evolution) in Table 6 below.

**[Table 5]**

| | | |
|---|---|---|
| coupling method | Amine coupling | |
| Sensor chip | HC1000M | |
| Running buffer | PBS pH 7.4 | |
| flow cell 1 | Activation | 200mM EDC, 100mM NHS |
| | Blocking protein | BSA |
| | Blocking | 1 M Ethanolamine*HCl |
| flow cell 2 | Activation | 200mM EDC, 100mM NHS |
| | Ligand concentration | 50 µg/ml in 5 mM acetate buffer (pH 4.0) |
| | Target RU | 5000 RU |
| | Blocking solution | 1 M Ethanolamine*HCl |

**[Table 6]**

| **2) Kinetic evaluation** | | | | |
|---|---|---|---|---|
| Running buffer | 1 X PBS | | | |
| analyte | name | Anti-ITGB2 antibody, TAMpep | | |
| | concentration | Anti-ITGB2 antibody | 1.5625,3 125, 6 25, 12 5.25, 50, 100n M | |
| | | TAMpep | 15.626, 31.25, 62.5, 125, 250, 500, 100 0 nM | |
| Binding setting | Contact time (min) | 2 min | flow rate (µl/min) | 30 µl/min |
| | Dissociation time (min) | 5 min | Stabilization time ( min) | 0 |
| regeneration | Regereration solution | Glycine pH 1.5 | flow rate (µl/min) | 30 µl/min |
| | Contact time (sec) | 45 sec | Stabilization time (min) | 2 min |

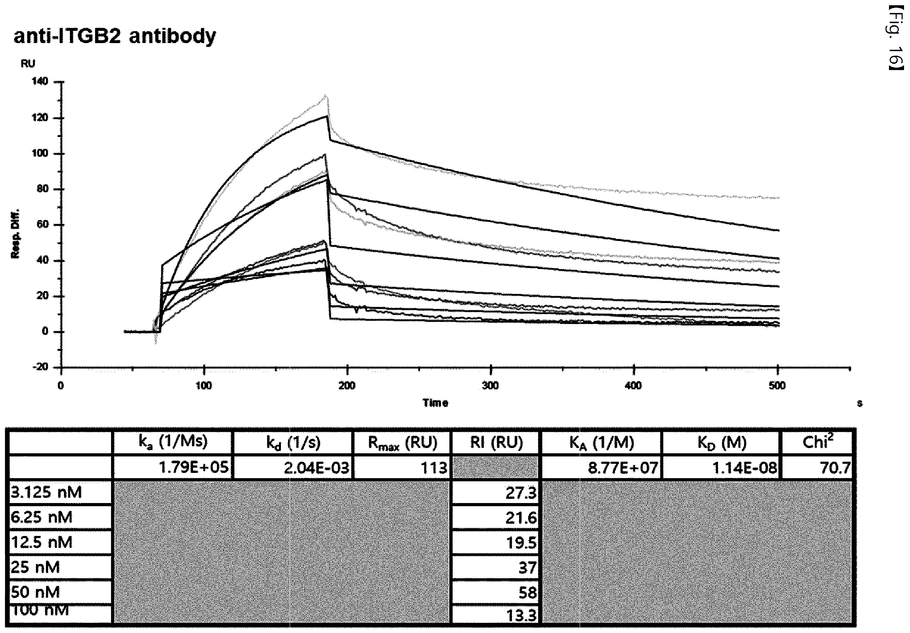

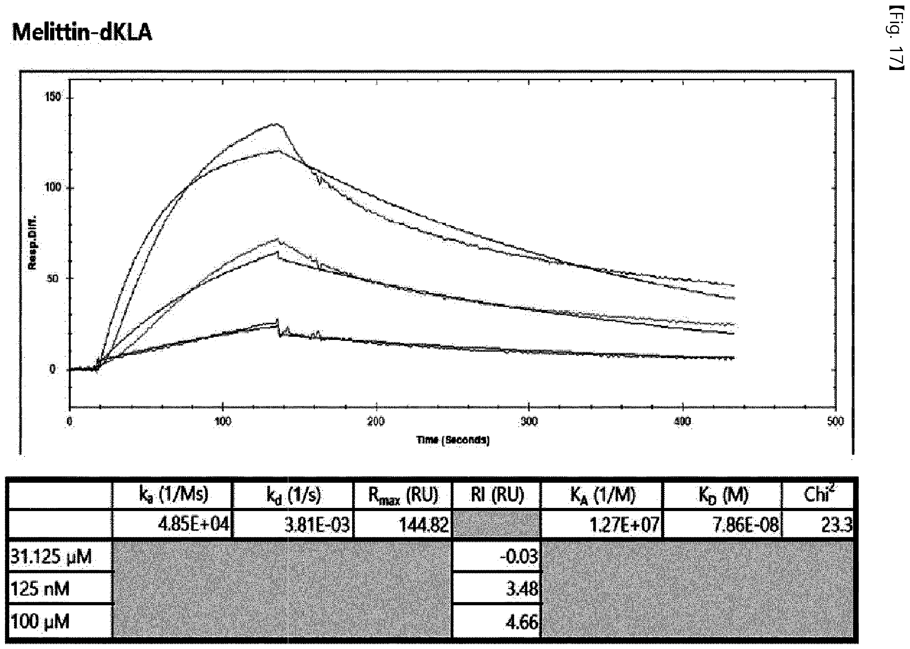

As a result, an equilibrium dissociation constant (KD), which was obtained by dividing a dissociation rate constant (kd) that evaluated the binding affinity between two molecules of a positive control ITGB2 antibody by an association rate constant (ka), was 1.14⁻⁸, and the equilibrium dissociation constant of Melittin-dKLA was 7.86⁻⁸ (FIGS. 16 and 17). Therefore, it was confirmed that Melittin-dKLA had binding affinity to ITGB2.

### Example 7. Confirmation of M2 TAM apoptotic effect through ITGB2 of PDC

### 7-1. Fabrication of cell model of ITGB2 expression suppression

As a cell model used to confirm whether apoptosis was induced through ITGB2 in M2 macrophages, a cell model in which the expression of ITGB2 was suppressed in M2 macrophages was fabricated using Crispr/cas9. Specifically, cells differentiated into M2 macrophages were cultured in an Opti-MEM medium. The Crispr/cas9 was mixed with Cas9 protein V2 (Thermo Fisher scientific) and gRNA (Genscript; ITGB2; Table 7) of a CRISPRMAX^{™} Reagent kit (Thermo Fisher scientific) and a Cas9 Plus^{™} Reagent (Thermo Fisher scientific), and a CRISPRMAX^{™} Reagent was diluted in the Opti-MEM medium, mixed together, and reacted for 5 to 10 minutes, and then added to cells differentiated into M2 macrophages and cultured at 37°C for 2 to 3 days. Thereafter, the mRNA expression level of ITGB2 was confirmed.

**[Table 7]**

| sgRNA | Sequences (5' to 3') |
|---|---|
| ITGB2 | mG*mU*mU*rCrArArCrGrUrGrArCrCrUrUrCrCrGrGrCrGrUrUrUrArG rArGrCrUrArGrArArArUrArGrCrArArGrUrUrArArArArUrArArGrGrCrUr ArGrUrCrCrGrUrUrArUrCrArArCrUrUrGrArArArArArGrUrGrGrCrArCrC rGrArGrUrCrGrGrUrGrCrU*mU*mU* mU |

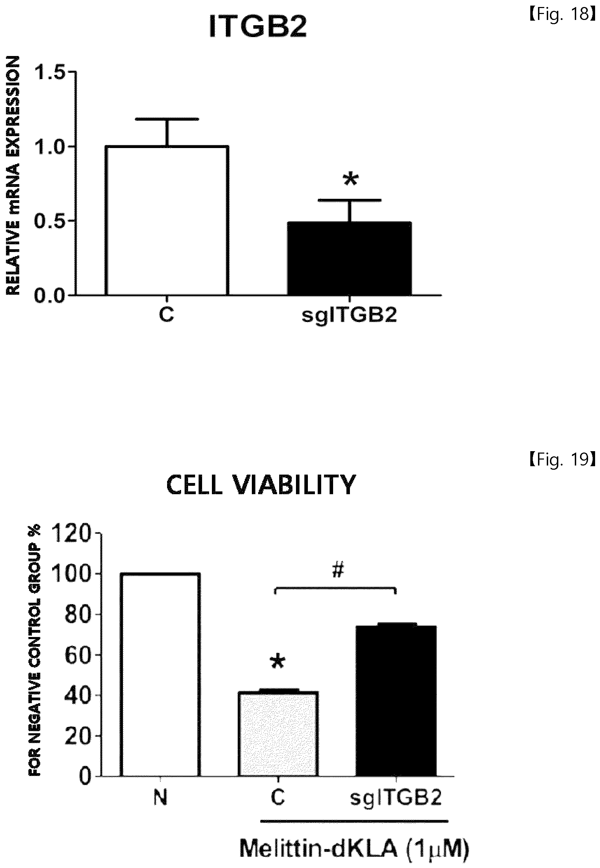

As a result, it was shown that in M2 macrophages injected with ITGB2 sgRNA, ITGB2 expression was significantly decreased compared to the control group (FIG. 18).

### 7-2. Confirmation of M2 TAM apoptosis through ITGB2 of Melittin-dKLA

To confirm whether Melittin-dKLA bound to ITGB2 to induce apoptosis in M2 macrophages, Melittin-dKLA (1 µM) was treated for 24 hours to M2 macrophages in which ITGB2 expression was suppressed by Crispr/cas9 fabricated in Example 7-1 above, and cell viability was analyzed using a CCK-8 assay. Specifically, Melittin-dKLA (1 µM) reacted with ITGB2 knockdown macrophages fabricated in Example 7-1 above for 1 hour. After the reaction, the medium was replaced and cultured at 37°C for 24 hours. To determine cell viability, a CCK-8 reagent (Enzo Life Sciences) was added in 1/10 of the medium and reacted at 37°C for 3 hours. Absorbance was measured at 450 nm using a microplate reader (Molecular Devices).

As a result, in control M2 macrophages, cell viability was significantly reduced by Melittin-dKLA, but in M2 macrophages in which ITGB2 was knocked down, the decrease in cell viability by Melittin-dKLA was suppressed (FIG. 19). Therefore, through this, it could be confirmed that Melittin-dKLA induced apoptosis of M2 macrophages by specifically binding to ITGB2 of M2 macrophages.

### 7-3. Confirmation of M2 TAM apoptosis through ITGB2 of TB511

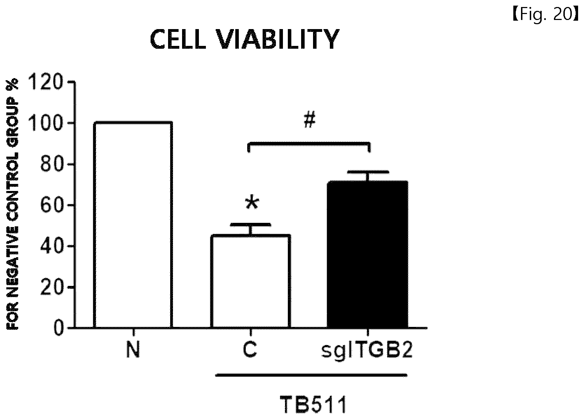

To confirm whether TB511 (TAMpep826-dKLA) induced apoptosis through ITGB2 in M2 macrophages, TB511 reacted with M2 macrophages in which ITGB2 expression was suppressed by Crispr/cas9 fabricated in Example 7-1 above. As a result, the M2 macrophages showed about 50% of cell viability by TB511, but the cell viability significantly increased in cells where ITGB2 expression was suppressed (FIG. 20).

### 7-4. Confirmation of reduced apoptosis induction of TB511 by ITGB2 antibody

To confirm whether TB511 induced apoptosis through ITGB2 in M2 macrophages, the M2 macrophages with suppressed ITGB2 expression fabricated in Example 7-1 above were pretreated with an anti-ITGB2 antibody (Polyclonal Rabbit anti-Human ITGB2/CD18 antibody) (LS-C312785; LS Bio) (1 µg) for 1 hour, and then reacted with TB511 (1 µM) for 1 hour. Thereafter, the induction of apoptosis was confirmed by cell viability analysis using the CCK-8 assay, protein expression of caspase-3, an apoptosis marker, was confirmed by Western blot analysis, gene expression of caspase-3, caspase-8, and caspase-9 was confirmed by Real-time PCR, and induction of protein expression of caspase-3 was confirmed by immunofluorescence.

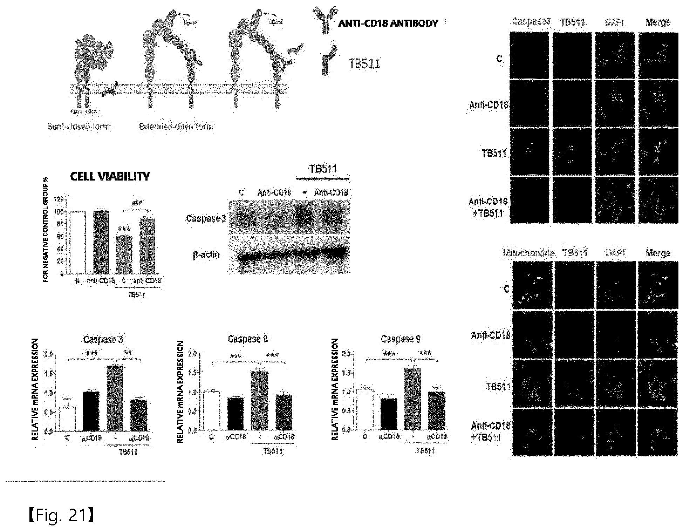

As a result, the M2 macrophages showed no effect on cell viability by the ITGB2 antibody, but cell viability was significantly reduced by TB511. On the other hand, in M2 macrophages pretreated with the ITGB2 antibody, the cell viability was significantly increased by TB511 (FIG. 21). In addition, caspase-3 expression was significantly increased by TB511 in M2 macrophages, whereas the caspase-3 expression was significantly decreased by TB511 in M2 macrophages pretreated with the ITGB2 antibody (FIG. 21). In addition, in M2 macrophages, the expression of caspase-3, caspase-8, and caspase-9 was significantly increased by TB511, whereas in M2 macrophages pretreated with the ITGB2 antibody, the expression of caspase-3, caspase-8, and caspase-9 was significantly decreased by TB511 (FIG. 21). In addition, in M2 macrophages, the caspase-3 expression was increased by TB511, whereas in M2 macrophages pretreated with the ITGB2 antibody, the caspase-3 expression was decreased by TB511 (FIG. 21). Additionally, as a result of confirming the mitochondrial targeting of TB511, in the M2 macrophages pretreated with ITGB2 antibody, co-localization of the mitochondria and TB511 was decreased compared to cells not pretreated with ITGB2 (FIG. 21).

### Example 8. Confirmation of efficacy of PDC in 3D cancer organoids

### 8-1. Confirmation of effect of TB511 in breast cancer 3D organoids

To complement the limitations *in vitro* and replace *in vivo,* a 3D organoid mimicking a tumor microenvironment (TME) of breast cancer was fabricated and an effect of TB511 thereon was confirmed. Specifically, an RPMI-1640 culture medium was mixed to contain 3% Matrigel, and THP-1 cells fabricated to express GFP using MDA-MB-231 human breast cancer cells, Cancer-associated Fibroblast (CAF), and Incucyte^{®}Nuclight Lentivirus Reagents (Sartorius) were counted and dispensed into a 96 u-bottom 3-D culture plate (Sbio) at a ratio of 5 : 1 : 1 to be 200 µl. After culturing, the cells were collected by centrifugation at 1200 rpm for 3 minutes and then cultured in a 37°C incubator for 48 hours to form spheroids. From day 2 of spheroid formation, TB511 was treated every three days at concentrations of 1 µM, 2 µM, 4 µM, and 8 µM, and images were taken using a fluorescence microscope. After taking bright-field images, the spheroid area was measured using a method for measuring a Ferret diameter. In addition, the average tumor spheroid diameter was measured using an open source, and the average diameter of three spheroids was analyzed with ImageJ (software version 1.47 m). The spheroid size was analyzed by taking bright-field images (20X), and then measuring the average tumor spheroid diameter of three spheroids with ImageJ (software version 1.47 m). Additionally, on day 10 of drug treatment, immunofluorescent staining was performed by fixing spheroids and reacting with an antibody (abcam) against ki-67, a tumor proliferation factor (control staining: DAPI). For THP-1, images of GFP fluorescence expressed within the cells were taken using a confocal microscope without performing separate staining, and the number of cells expressing fluorescence was measured.

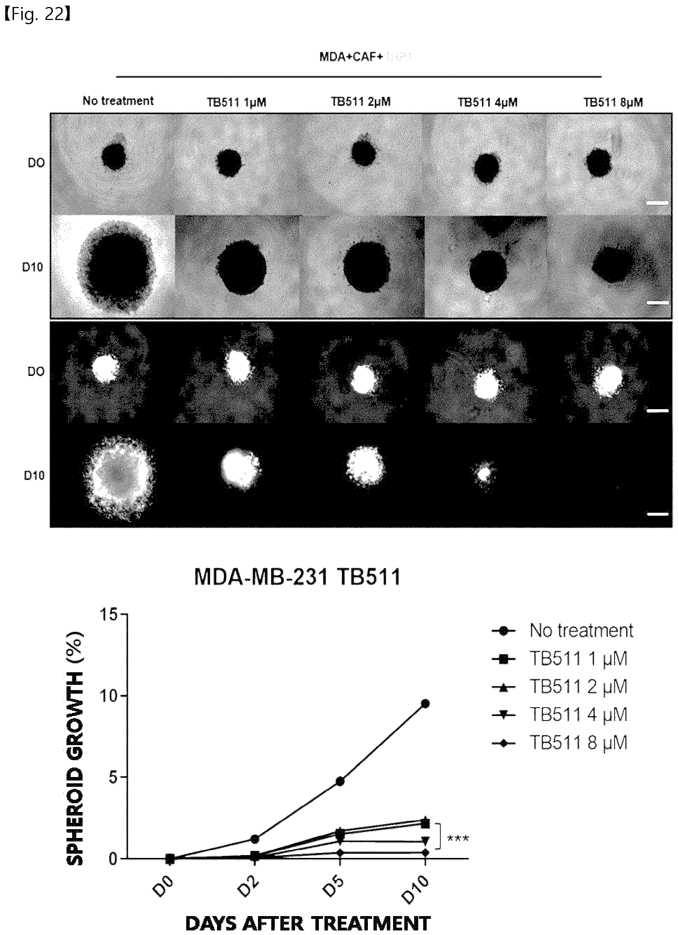

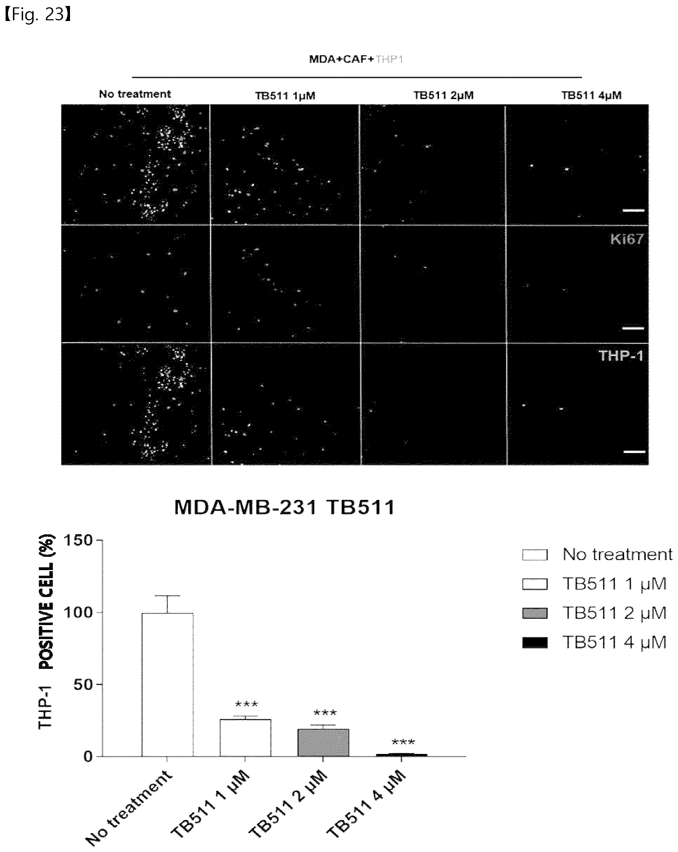

As a result, it was shown that in a group that was not treated with a drug, the size of the spheroids gradually increased over time, and thus THP-1 cells, which were monocytes, differentiated into TAMs by cancer cells and TME when co-cultured with cancer cells and CAFs, thereby promoting tumor growth. On the other hand, in a group treated with TB511, it was confirmed that the size of spheroids decreased in a concentration-dependent manner from a concentration of 1 µM on day 10 (FIG. 22), and it was shown that the number of THP-1 cells expressing GFP fluorescence was significantly decreased in a concentration-dependent manner compared to a druguntreated group (***p < 0.001) (FIG. 23). Thus, it was confirmed that TB511, an ITGB2-binding PDC, selectively bound to THP-1 cells and induced apoptosis of MDA-MB-231 human breast cancer.

### 8-2. Confirmation of effect of TB511 in lung cancer 3D organoids

Lung cancer 3D spheroids were fabricated by mixing A549 human lung cancer cells, CAFs, and THP-1 cells using the method of Example 8-1 above, and then treated with TB511 at concentrations of 1 µM, 2 µM, and 4 µM, respectively, and the effects thereof were analyzed as in Example 8-1 above. When fabricating the spheroids, 3D spheroids were also fabricated by mixing cancer cells alone or only cancer cells + CAF cells, and the sizes of the spheroids were compared.

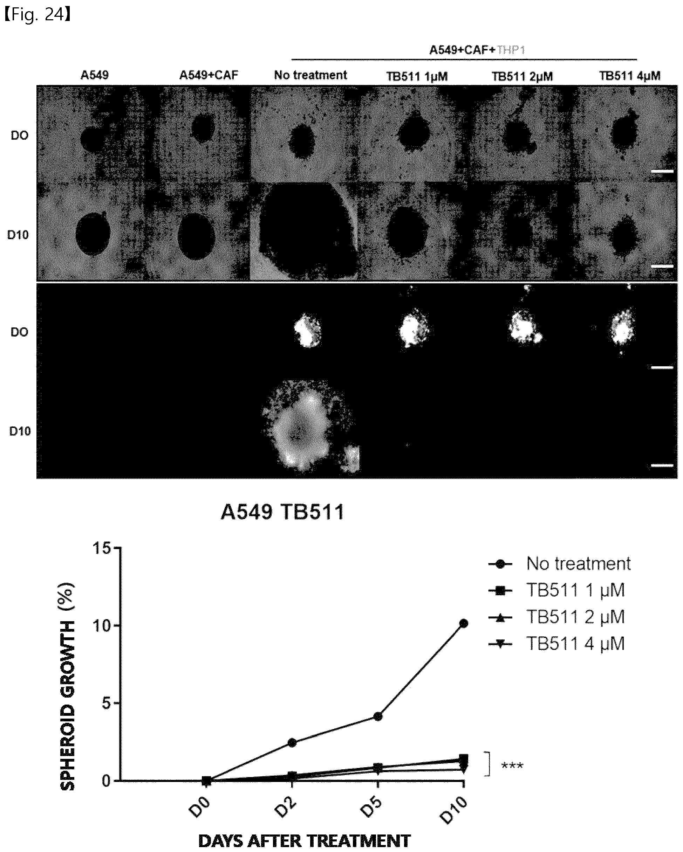

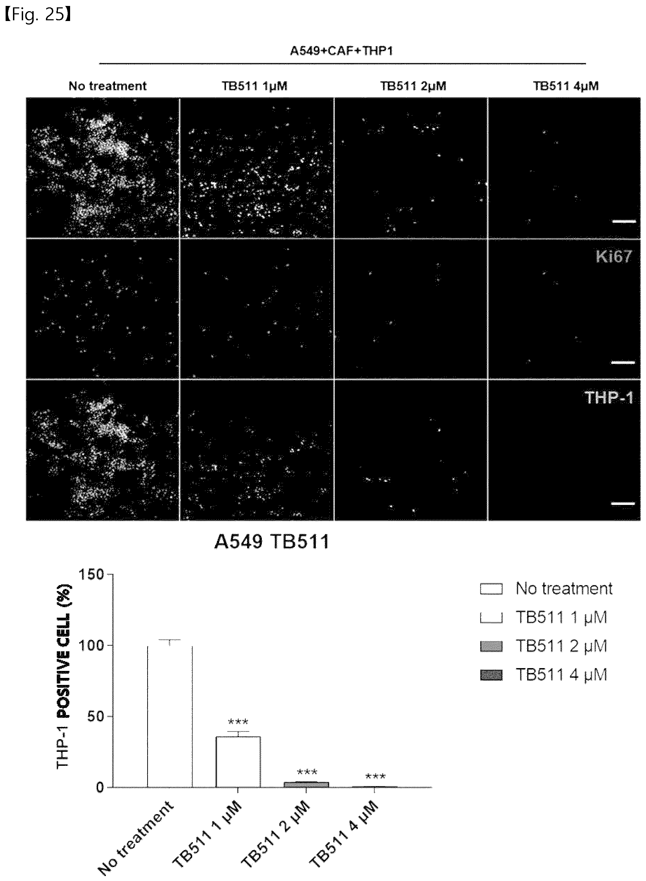

As a result, it was shown that the size of the spheroids was significantly increased when spheroids were formed with A549+CAF+THP-1 cells compared to when spheroids were formed with A549 cells alone or only A549 + CAF cells. In addition, the size of spheroids in a TB511-treated group was significantly reduced in a concentration-dependent manner compared to a group not treated with TB511 (****p* < 0.001) (FIG. 24). In addition, as a result of confirming related markers by fluorescently staining the spheroids on day 10 after drug treatment, compared to the group not treated with TB511, the number of THP-1 cells was significantly decreased in a concentration-dependent manner, and a tumor proliferation factor ki-67 was also decreased in a concentration-dependent manner (***p < 0.001) (FIG. 25).

### 8-3. Confirmation of effect of M-DM1 in lung cancer 3D organoids

In order to confirm the efficacy of M-DM1 (Mel-DM1) on lung cancer, lung cancer 3D spheroids were fabricated by mixing A549 human lung cancer cells, CAFs, and THP-1 cells using the method of Example 8-1 above, and then treated with M-DM1 at concentrations of 1 µM, 2 µM, and 4 µM, respectively, and the effects thereof were analyzed as in Example 8-1 above.

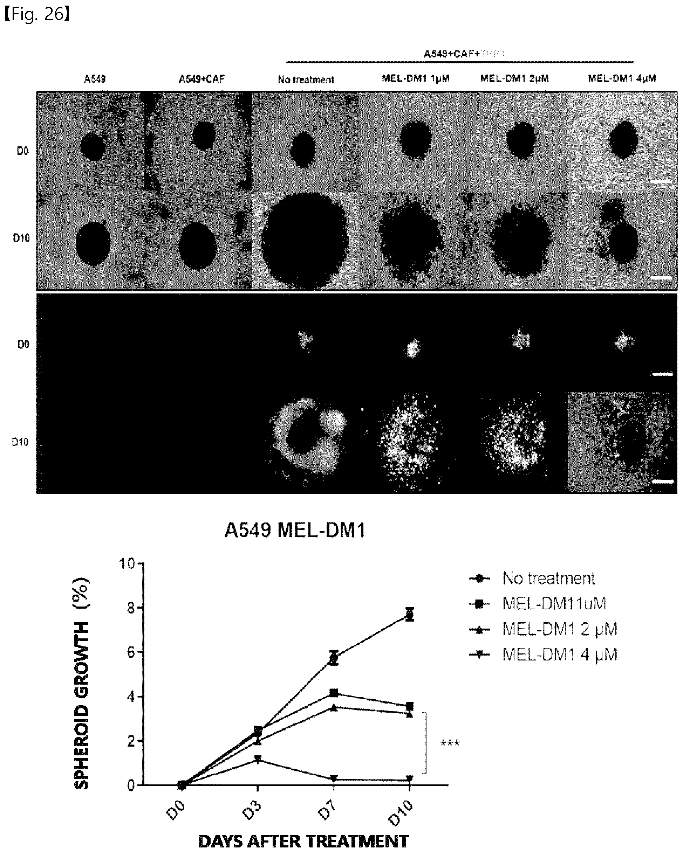

As a result, it was shown that the size of spheroids was decreased in a concentration-dependent manner in a group treated with M-DM1 compared to a group not treated with M-DM1 (***p < 0.001) (FIG. 26).

### Example 9. Confirmation of efficacy of PDC in anticancer drug-resistant 3D cancer organoids

### 9-1. Confirmation of effect of TB511 in anticancer drug-resistant lung cancer 3D organoids

In order to confirm the efficacy of TB511 on Carboplatin-resistant lung cancer, 3D spheroids were formed by mixing A549 cells, CAFs, and THP-1 cells using the method of Example 8-1 above, and then treated with TB511 at concentrations of 1 µM, 2 µM, and 4 µM, respectively, and the effects thereof were analyzed as in Example 8-1 above.

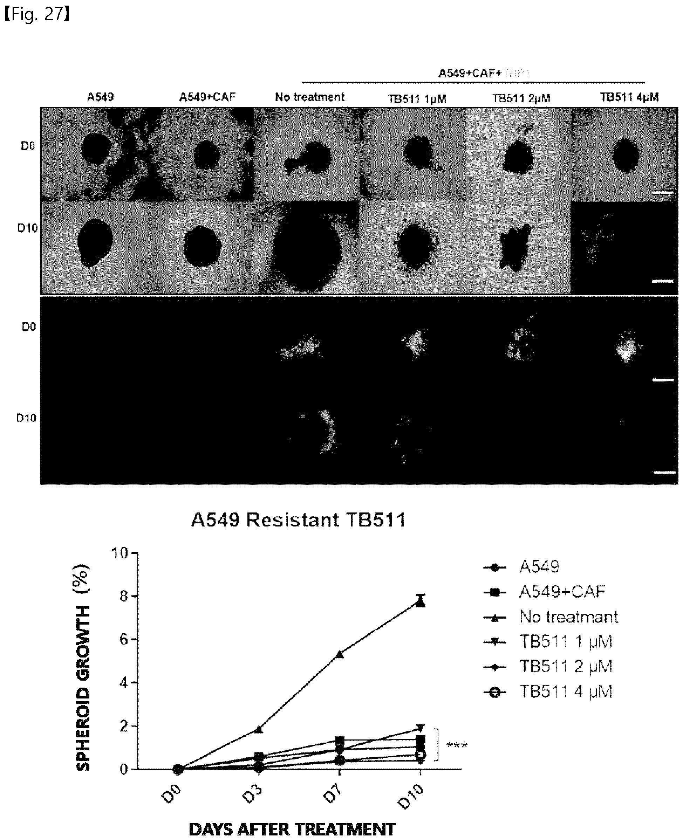

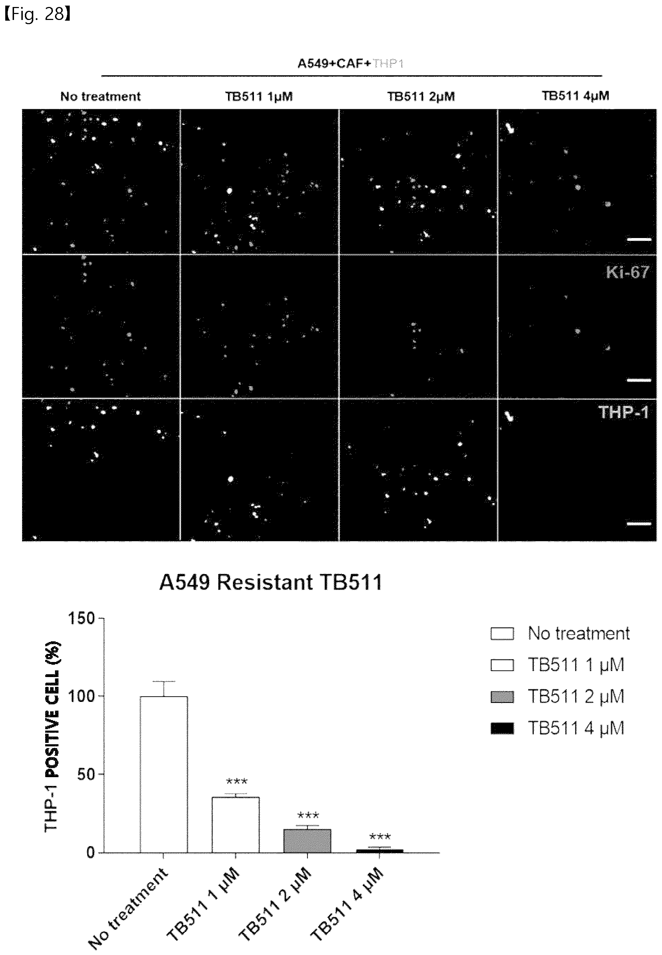

As a result, the size of the spheroids when the spheroids were formed with A549+CAF+THP-1 cells was significantly increased compared to when the spheroids were formed with A549-resistant cells alone or only A549+CAF cells, and the size of the spheroids in a TB511-treated group was significantly reduced in a TB511 concentration-dependent manner compared to a group not treated with TB511 (****p* < 0.001) (FIG. 27). In addition, as a result of confirming related markers by staining the spheroids with immunofluorescence on day 10 after drug treatment, the number of THP-1 cells in the TB511-treated group was significantly decreased in a concentration-dependent manner compared to the TB511-untreated group, and the tumor proliferation factor ki-67 was also significantly decreased from 2 µM (***p < 0.001) (FIG. 28).

### 9-2. Confirmation of effect of TB511 in anticancer drug-resistant prostate cancer 3D organoids

In order to confirm the efficacy of TB511 on Docetaxel-resistant prostate cancer, 3D spheroids were formed by mixing Docetaxel-resistant PC3 prostate cancer cells, CAFs, and THP-1 cells using the method of Example 8-1 above, and then treated with TB511 at concentrations of 1 µM, 2 µM, and 4 µM, respectively, and the effects thereof were analyzed as in Example 8-1 above.

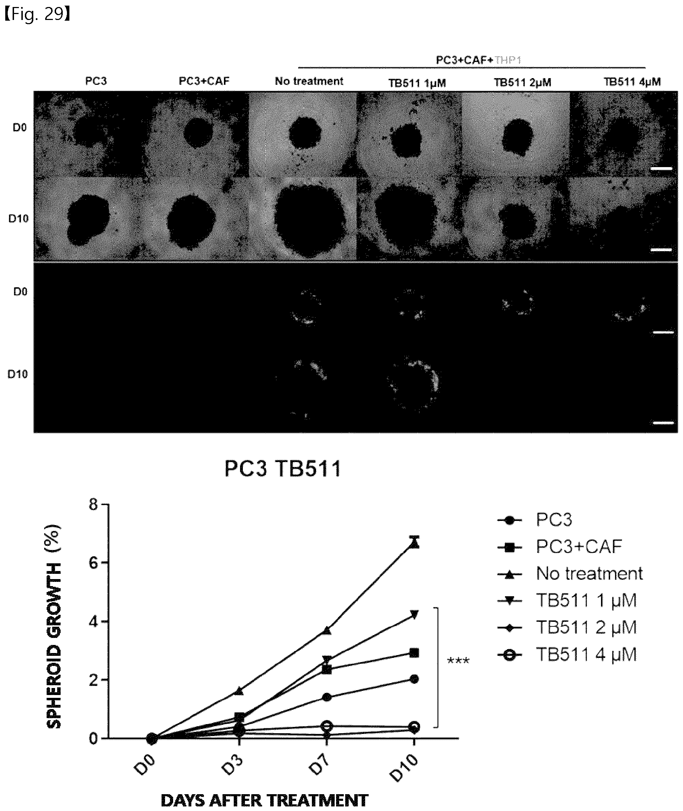

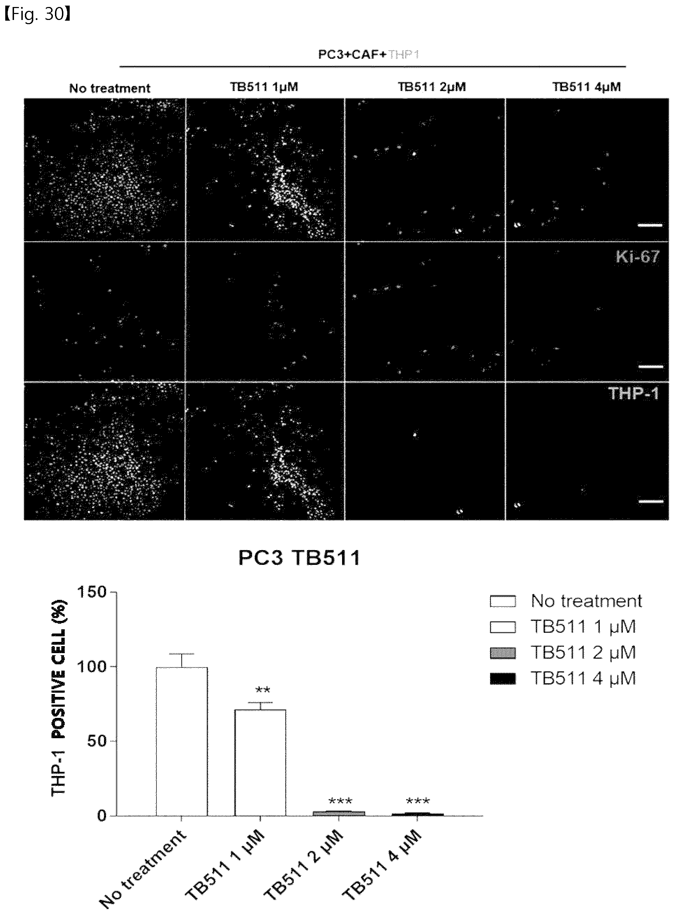

As a result, the size of the spheroids when the spheroids were formed with PC3+CAF+THP-1 cells was significantly increased compared to when the spheroids were formed with PC3-resistant cells alone or only PC3+CAF cells, and the size of the spheroids in a TB511-treated group was significantly reduced in a TB511 concentration-dependent manner compared to a group not treated with TB511 (***p < 0.001) (FIG. 29). In addition, as a result of confirming related markers by staining the spheroids with immunofluorescence on day 10 after drug treatment, the number of THP-1 cells in the TB511-treated group was significantly decreased in a concentration-dependent manner compared to the TB511-untreated group, and the tumor proliferation factor ki-67 tended to be reduced (***p* < 0.01, ****p* < 0.001) (FIG. 30).

### Example 10. Confirmation of efficacy of ADC in 3D cancer organoids

### 10-1. Confirmation of effect of ADC in breast cancer 3D organoids

In order to confirm the efficacy of an ITGB2-binding antibody-drug conjugate (ADC) on breast cancer, 3D spheroids were formed by mixing MDA-MB-231 human breast cancer cells, CAFs and THP-1 cells using the method of Example 8-1 above, and CD18-DM1 or CD18-MMAE, which was ADC of the present disclosure, was treated at concentrations of 10 µg and 20 µg, respectively, and the effects were analyzed as in Example 8-1 above.

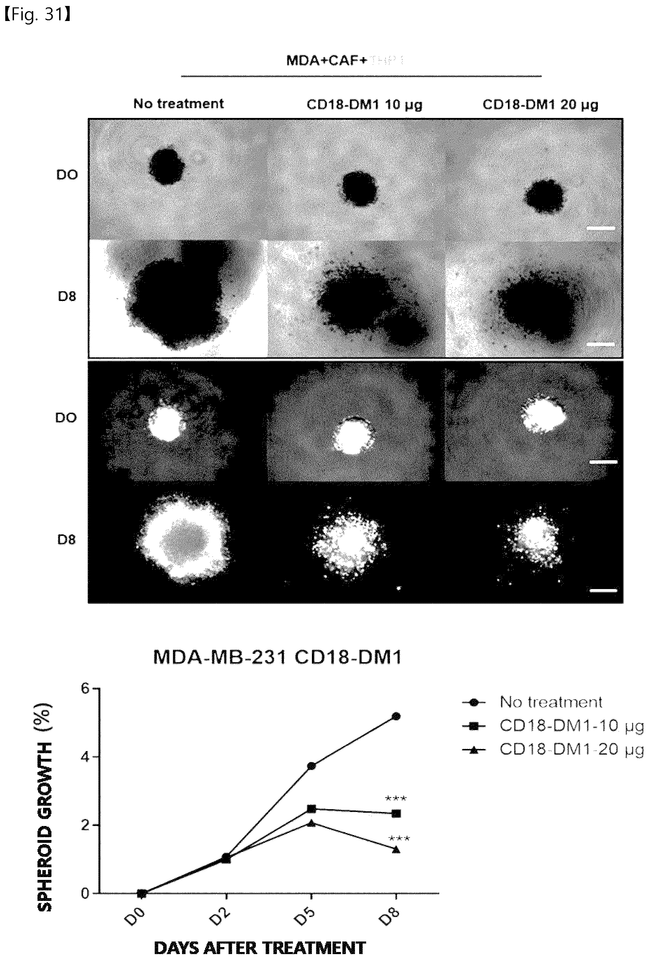

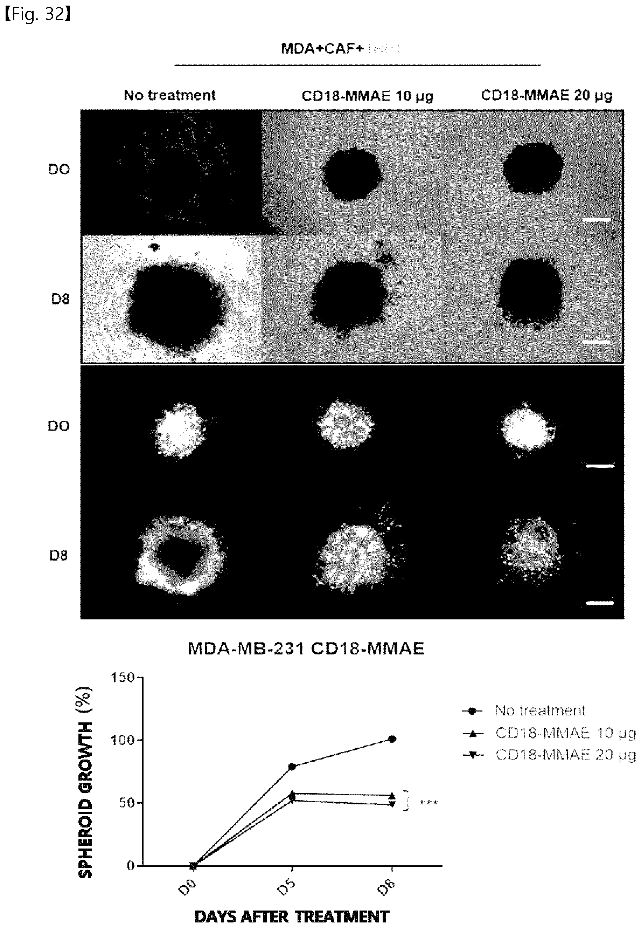

As a result, in a group not treated with ADC, the size of breast cancer spheroids was gradually increased over time, but in a group treated with ADC, the size of spheroids was decreased on day 8 (FIGS. 31 and 32). In addition, it was shown that GFP-positive THP-1 cells were strongly increased in the group not treated with ADC, but were decreased in a concentration-dependent manner in a CD18-DM1 or CD18-MMAE treated group (***p < 0.001) (FIGS. 31 and 32).

### 10-2. Confirmation of effect of ADC in lung cancer 3D organoids

In order to confirm the efficacy of an ADC on lung cancer, 3D spheroids were formed by mixing A549 human lung cancer cells, CAFs and THP-1 cells using the method of Example 8-1 above, and CD18-DM1 or CD18-MMAE, which was ADC of the present disclosure, was treated at a concentration of 10 µg, respectively, and the effects were analyzed as in Example 8-1 above.

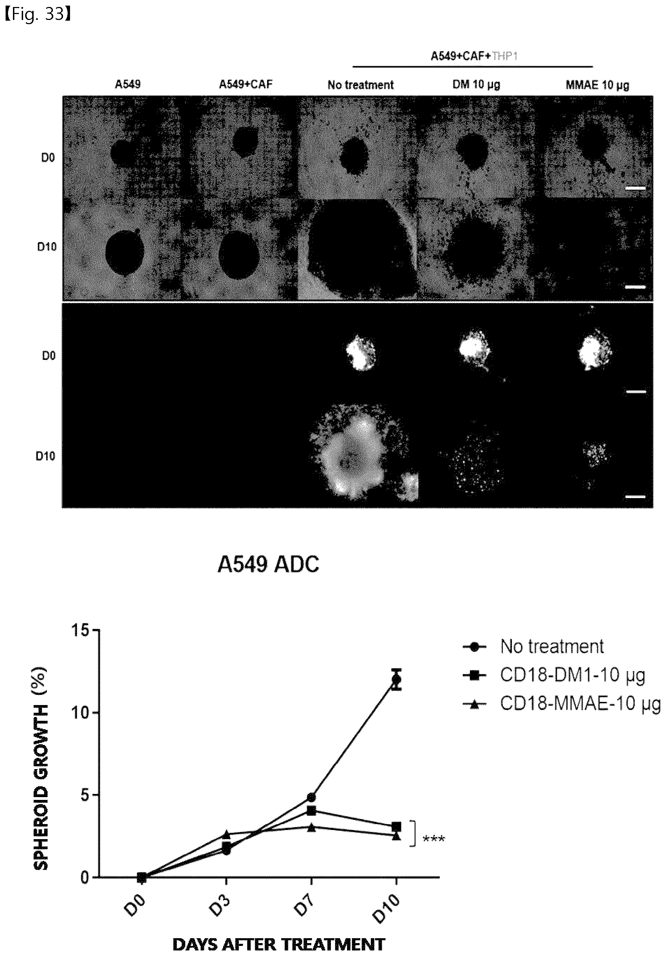

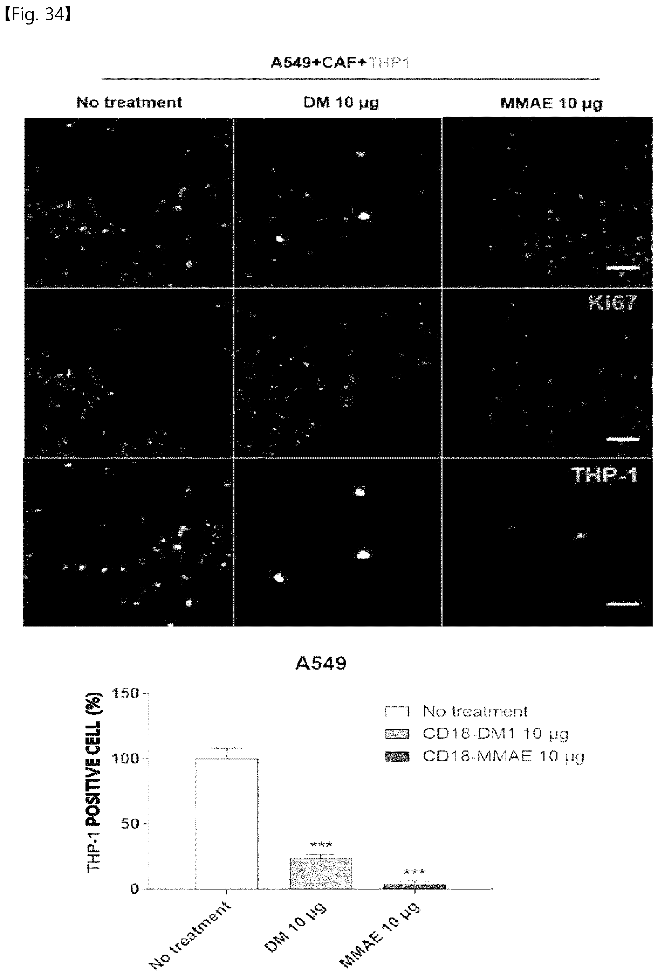

As a result, it was shown that in the group not treated with ADC, the size of spheroids was gradually increased over time, whereas in the group treated with CD18-DM1 or CD18-MMAE, the size of spheroids was decreased on day 8 (FIG. 33). In addition, as a result of confirming related markers by staining the spheroids with immunofluorescence on day 8 after drug treatment, it was found that THP-1 cells were strongly increased in the group not treated with ADC, whereas the THP-1 cells were significantly decreased in the ADC-treated group (****p* < 0.001) (FIG. 34).

### Example 11. Confirmation of efficacy of ADC in anticancer drug-resistant 3D cancer organoids

### 11-1. Confirmation of effect of ADC in anticancer drug-resistant lung cancer 3D organoids

In order to confirm the efficacy of ADC on Carboplatin-resistant lung cancer, 3D spheroids were formed by mixing Carboplatin-resistant A549 cells, CAFs, and THP-1 cells using the method of Example 8-1 above, and then treated with CD18-DM1 or CD18-MMAE at a concentration of 10 µg, respectively, and the effects thereof were analyzed as in Example 8-1 above.

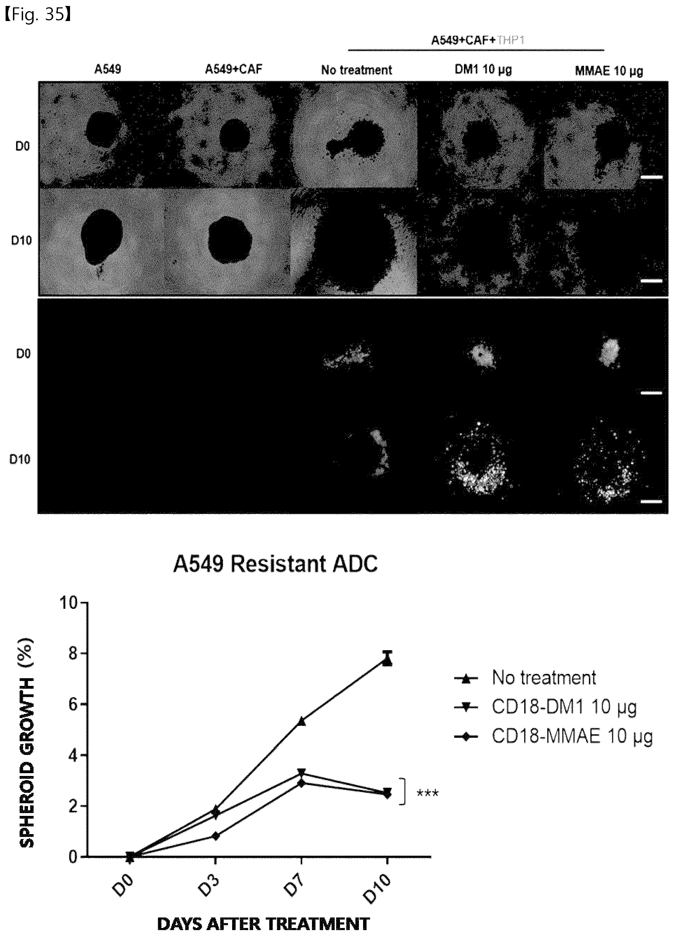

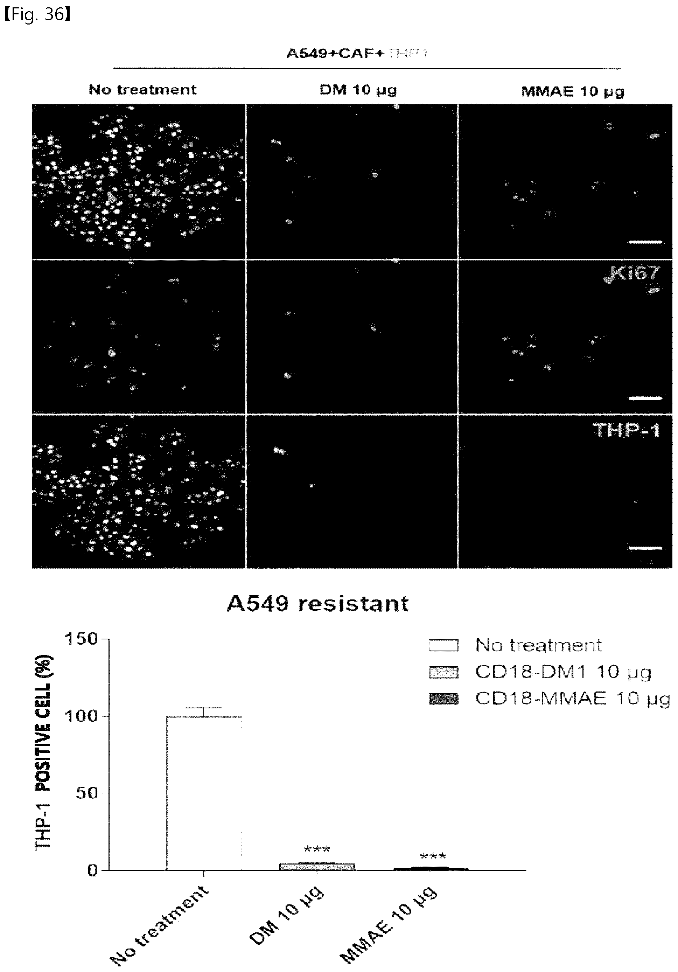

As a result, it was shown that in the group not treated with ADC, the size of spheroids was gradually increased over time, whereas in the group treated with CD18-DM1 or CD18-MMAE, the size of spheroids was decreased on day 8 (FIG. 35). In addition, as a result of confirming related markers by staining the spheroids with immunofluorescence on day 8 after drug treatment, it was shown that compared to the group not treated with ADC, in the group treated with CD18-DM1 or CD18-MMAE, the number of THP-1 cells was significantly decreased and the tumor proliferation factor ki-67 was also decreased (****p* < 0.001) (FIG. 36).

### 11-2. Confirmation of effect of ADC in anticancer drug-resistant prostate cancer 3D organoids

In order to confirm the efficacy of ADC on Docetaxel-resistant prostate cancer, 3D spheroids were formed by mixing Docetaxel-resistant PC3 prostate cancer cells, CAFs, and THP-1 cells using the method of Example 8-1 above, and then treated with CD18-DM1 or CD18-MMAE at a concentration of 10 µg, respectively, and the effects thereof were analyzed as in Example 8-1 above.

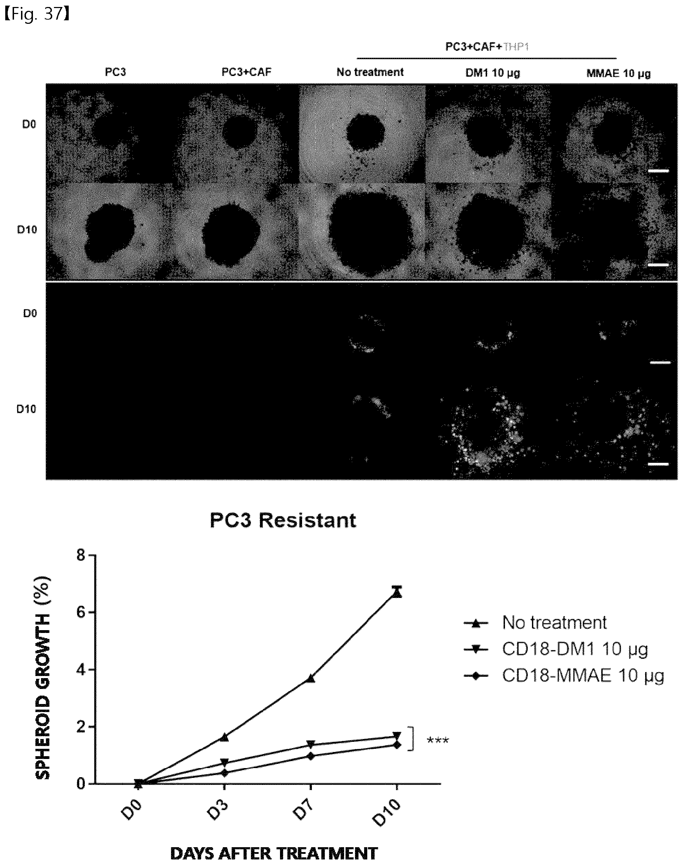

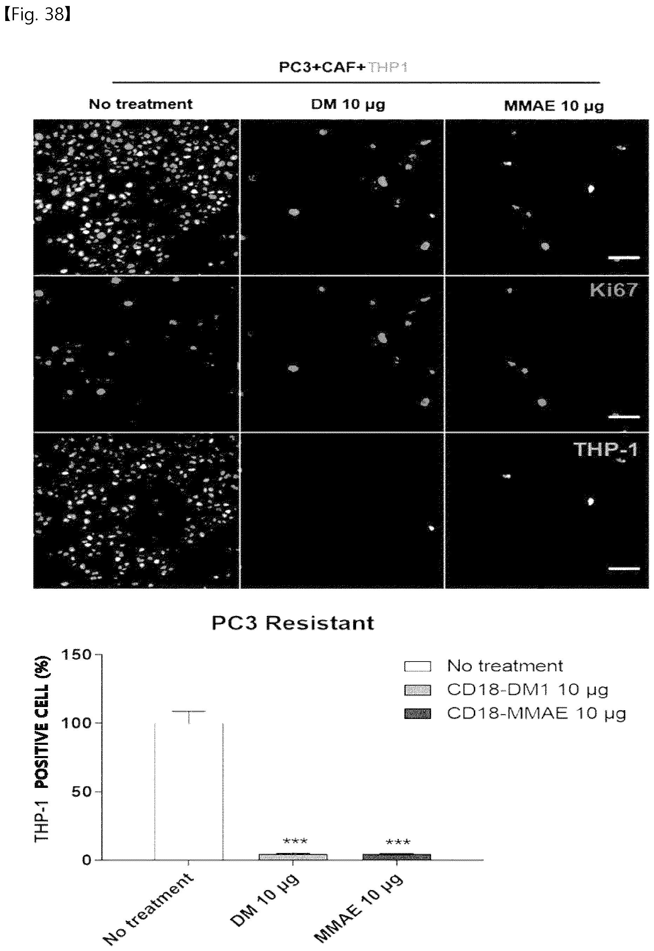

As a result, it was shown that in the group not treated with ADC, the size of spheroids was gradually increased over time, whereas in the group treated with CD18-DM1 or CD18-MMAE, the size of spheroids was decreased on day 8 (FIG. 37). In addition, as a result of confirming related markers by staining the spheroids with immunofluorescence on day 8 after drug treatment, it was shown that the fluorescence was strongly increased in the group not treated with ADC, whereas in the group treated with CD18-DM1 or CD18-MMAE, the number of THP-1 cells was significantly reduced, and the tumor proliferation factor ki-67 was also decreased (***p < 0.001) (FIG. 38).

## Claims

1. A drug delivery conjugate comprising an ITGB2-binding moiety; and a drug binding to the ITGB2-binding moiety.

2. The drug delivery conjugate of claim 1, wherein the ITGB2-binding moiety is a small molecule, a virus, an antibody, an antibody fragment, an aptamer, a hormone, a cytokine, a chemokine, a ligand, a peptide that is a portion of a cytokine, or a peptide that is a portion of a ligand, that specifically binds to the ITGB2.

3. The drug delivery conjugate of claim 1, wherein the ITGB2-binding moiety specifically binds to an active conformation in which CD18 in an extended.

4. The drug delivery conjugate of claim 1, wherein the ITGB2 includes an amino acid sequence represented by SEQ ID NO: 7 or 8.

5. The drug delivery conjugate of claim 1, wherein the ITGB2-binding moiety binds to amino acids at positions 449 to 617 of ITGB2.

6. The drug delivery conjugate of claim 5, wherein the ITGB2-binding moiety includes an amino acid sequence represented by SEQ ID NO: 1 or 2.

7. The drug delivery conjugate of claim 5, wherein the drug delivery conjugate includes a peptide including an amino acid sequence represented by SEQ ID NO: 5 or 6.

8. The drug delivery conjugate of claim 1, wherein the ITGB2-binding moiety binds to amino acids at positions 449 to 617 of ITGB2.

9. The drug delivery conjugate of claim 1, wherein the ITGB2-binding moiety binds to amino acids at positions 732 to 748 of ITGB2, amino acids at positions 504 to 508, amino acids at positions 534 to 546, amino acids at positions 449 to 617, or amino acids at positions 23 to 700.

10. The drug delivery conjugate of claim 1, wherein the ITGB2-binding moiety includes an anti-CD18 antibody or an immunologically active fragment thereof.

11. The drug delivery conjugate of claim 10, wherein the immunologically active fragment is any one selected from the group consisting of Fab, Fd, Fab', dAb, F(ab'), F(ab')₂, a single chain fragment variable (scFv), Fv, a single chain antibody, a Fv dimmer, a complementarity determining region fragment, a humanized antibody, a chimeric antibody, and a diabody.

12. The drug delivery conjugate of claim 1, wherein the drug is a compound, RNA, DNA, an antibody, an effector, a prodrug, a toxin, a peptide, or a radionuclide.

13. The drug delivery conjugate of claim 1, wherein the drug is immunogenic apoptosis-inducing agents, pro-apoptotic peptides, microtubulin structure formation inhibitors, meiosis inhibitors, topoisomerase inhibitors, DNA intercalators, toxins or anticancer agents.

14. The drug delivery conjugate of claim 13, wherein the pro-apoptotic peptide is selected from the group consisting of KLA, alpha-defensin-1, BMAP-28, Brevenin-2R, Buforin IIb, cecropin A-Magainin 2 (CA-MA-2), Cecropin A, Cecropin B, chrysophsin-1, D-K6L9, Gomesin, Lactoferricin B, LLL27, LTX-315, Magainin 2, Magainin II-bombesin conjugate (MG2B), Pardaxin, and combinations thereof.

15. The drug delivery conjugate of claim 13, wherein the immunogenic apoptosis-inducing agent is selected from the group consisting of an anthracycline-based anticancer agent, a taxane-based anticancer agent, an anti-EGFR antibody, a BK channel agonist, bortezomib, cardiac glycoside, cyclophosmide-based anticancer agents, a GADD34/PP1 inhibitor, LV-tSMAC, Measles virus, bleomycin, mitoxantrone, oxaliplatin, and combinations thereof.

16. The drug delivery conjugate of claim 13, wherein the anticancer agent is selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), Streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof.

17. The drug delivery conjugate of claim 1, wherein the drug delivery conjugate specifically delivers a drug to M2 tumor-associated macrophages.

18. A peptide-drug conjugate (PDC) comprising an ITGB2-binding moiety including melittin, a variant thereof or analogues thereof; and a drug binding to the ITGB2-binding moiety.

19. The peptide-drug conjugate of claim 18, wherein the peptide-drug conjugate binds to amino acids at positions 466 to 565 of ITGB2.

20. The peptide-drug conjugate of claim 18, wherein the peptide-drug conjugate binds to LEU(528), THR(538), LEU(556), CYS(557), PHE(558), GLU(466), ILE(469), CYS(470) or ARG(471) of ITGB2.

21. An antibody-drug conjugate (ADC) comprising an antibody or immunologically active fragment thereof that binds to ITGB2; and a drug binding to the antibody or immunologically active fragment thereof.

22. The antibody-drug conjugate of claim 21, wherein the antibody-drug conjugate binds to an epitope including amino acids at positions 732 to 748 of ITGB2, an epitope including amino acids at positions 504 to 508, an epitope including amino acids at positions 534 to 546, an epitope including amino acids at positions 449 to 617, or an epitope including amino acids at positions 23 to 700.

23. The antibody-drug conjugate of claim 21, further comprising:
an antibody-drug conjugate linker.

24. The antibody-drug conjugate of claim 23, wherein the ADC linker is 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl) cyclohexane-1 carboxylate (SMCC), valine-citrulline-p-aminobenzyloxycarbonyl (val-cit-PAB), or N-succinimidyl (4-iodo-acetyl) aminobenzoate (STAB).

25. A pharmaceutical composition for preventing or treating cancer, comprising the drug delivery conjugate of claim 1, the peptide-drug conjugate of claim 18, or the antibody-drug conjugate of claim 21 as an active ingredient.

26. The pharmaceutical composition for preventing or treating cancer of claim 25, wherein the pharmaceutical composition is administered to a patient in whom the expression of ITGB2 is upregulated in M2 macrophages compared to M0 macrophages and M1 macrophages.

27. A method for screening a substance that specifically delivers a drug to M2 tumor-associated macrophages, comprising treating Integrin beta 2 (ITGB2) with a candidate substance; and selecting the candidate substance that binds to ITGB2.

28. The method of claim 27, wherein a candidate substance that specifically binds to CD18 in an extended active conformation is selected.

29. A method for selecting a patient having responsiveness to an anticancer agent targeting M2 macrophages, comprising determining the expression level of ITGB2 in an isolated sample.

30. The method of claim 29, further comprising:
determining a patient as having responsiveness to an anticancer agent targeting M2 macrophages, when the expression level of ITGB2 in M2 macrophages is higher than that in M0 macrophages and M1 macrophages.

31. A method for treating cancer, comprising administering to a subject suffering from cancer a pharmaceutically effective amount of the drug delivery conjugate of claim 1, the peptide-drug conjugate of claim 18, or the antibody-drug conjugate of claim 21.

32. A use of a drug delivery conjugate used for the preparation of a pharmaceutical composition for preventing and treating cancer.
